# EUROPEAN PATENT APPLICATION

(11) **EP 4 534 564 A1**
(43) Date of publication of application: **09.04.2025**
(21) Application number: 23815201.1
(22) Date of filing: 30.05.2023
(51) Int. Cl.: C07K 19/00, C07K 16/28, C12N 15/62, C12N 15/63, A61K 38/17, A61K 39/395, A61P 1/16, A61P 27/02, A61P 35/00

(54) **RECOMBINANT FUSION PROTEIN TARGETING PD-L1 AND VEGF, AND PREPARATION AND USE THEREOF**

(30) Priority: 31.05.2022 CN 202210639281
(71) Applicant: ImmuneOnco Biopharmaceuticals (Shanghai) Inc., Shanghai 201203 (CN)
(72) Inventor: TIAN, Wenzhi, Shanghai 201203 (CN); LI, Song, Shanghai 201203 (CN); CHEN, Dianze, Shanghai 201203 (CN); YANG, Chunmei, Shanghai 201203 (CN); ZHU, Wenqi, Shanghai 201203 (CN)
(74) Representative: Krauss, Jan
(86) International application number: PCT/CN2023/097088
(87) International publication number: WO 2023/232022

(57) **Abstract**

The application provides a recombinant fusion protein containing 1) an anti-PD-L1 antibody or an antigen-binding fragment thereof, and 2) an VEGF binding peptide, wherein the N-terminus of the heavy chain or the light chain of the anti-PD-L1 antibody or the antigen-binding fragment thereof is linked, via a linker, to the VEGF binding peptide. Also provided is a nucleic acid molecule for encoding the recombinant fusion protein, an expression vector containing the nucleic acid molecule, a method for preparing the recombinant fusion protein, and a method for treating a disease associated with PD-L1 and/or VEGF signaling using the recombinant fusion protein.

## Description

The application claims priority to Chinese patent application CN202210639281.X filed on May 31, 2022.

### FIELD OF THE INVENTION

The application relates to a recombinant fusion protein that targets PD-L1, VEGF and/or FcR, and its preparation and uses, especially its use in tumor treatment.

### BACKGROUND OF THE INVENTION

Cancer cells have developed several mechanisms to evade host cells' immune surveillance. For example, many tumor or cancer cells express on their surfaces a high level of PD-Ll and PD-L2, both of which bind to PD-1 on the surface of T cells, inducing T cell apoptosis.

In addition, cancer cell growth depends on sufficient supply of nutrition. Cancer cells themselves can secrete factors that promote blood vessel growth, such as vascular epithelial growth factors (VEGF). Inhibition of the activity of VEGF or its receptor(s) will stop blood supply to the solid tumor, thereby inhibiting its growth.

### PD-L1 and PD-1

PD-1/PD-L1 is one of the most studied inhibitory immune checkpoints.

PD-L1, also known as programmed death-ligand 1 or CD274, is a transmembrane protein that plays important roles in suppressing the immune system during some particular events such as tissue allografts, autoimmune disease and cancer development. In the tumor microenvironment (TME), the CD4⁺ Th1 helper cells and CD8⁺ T cells produce interferon-γ (IFN-γ), which on one hand promotes macrophages to kill tumors, and on the other hand induces PD-L1 expression by macrophages and tumor cells. The PD-1 on tumor-specific CD8⁺ T cells binds to the PD-L1 expressed by various cells in the TME, leading to CD8⁺ T cell anergy (Topalian, S. et al., (2016) Nat Rev Cancer 16(5): 275-287). The PD-1 may also bind to PD-L2, down-regulating the immune system.

An analysis of 196 tumor specimens from patients with renal cell carcinoma showed that tumors of high PD-L1 expression were associated with increased tumor aggressiveness and a 4.5-fold increase in death risk (Thompson RH, et al., (2004) PNAS. 101(49):17174-17179). About 1/5 to 1/4 patients with non-small cell lung cancer, melanoma or renal cell cancer had objective responses to BMS-936558, an anti-PD-1 antibody (SuzanneL. Topalian, et al., (2012) N Engl J Med 366:2443-2454).

### VEGF and VEGFR

VEGF is a pleiotropic growth factor that is central to tissue/wound repair programs, and is classified into VEGF-A (also known as VEGF165), VEGF-B, VEGF-C, VEGF-D, VEGF-E and PIGF. During the tissue healing process, VEGF, especially VEGF-A, drives formation of new blood vessels while down-regulating immunity (Canic M, et al., (2009) J Surg Res. 153:347-358; Leung DW, et al., (1989) Science. 246:1306-1309; Voron T, et al., (2014) Front Oncol. 4:70). Both of these properties of VEGF are key to the oncogenic process, as the newly formed tumor blood vessels may supply blood and nutrients to the tumor tissues, and the inhibition of anti-tumor immunity may promote tumor cell growth (Hanahan D, Weinberg RA. (2011) Cell. 144:646-674). In particular, VEGF is thought to exert its immune-suppressive effects via three main mechanisms: inhibiting dendritic cell (DC) maturation, reducing T cell infiltration, and increasing inhibitory cells in tumor microenvironment. Abnormal VEGF expression also contributes to generation of new blood vessels that are abnormal in structure and likely to leak, causing e.g., diabetic retinopathy, wet form age-related macular degeneration, and the like. Bevacizumab (Avastin), an FDA approved anti-VEGF monoclonal antibody drug, functions to treat cancers (colon cancer, lung cancer), non-squamous non-small cell lung cancer, renal cell cancer, glioblastoma multiforme, ovarian cancer and cervical cancer, by inhibiting the biological activity of VEGF. Another protein drug targeting VEGF, Aflibercept, was approved in United State and Europe for treatment of wet macular degeneration (tradename being Eylea), and for metastatic colorectal cancer (Zaltrap).

VEGF receptor (VEGFR) has three subtypes, VEGFR-1, VEGFR-2 and VEGFR-3, all having an extracellular portion consisting of 7 immunoglobulin-like domains, a transmembrane region and an intracellular portion. VEGFR-2 appears to mediate almost all of the known cellular responses to VEGF, while VEGFR-1 sequesters VEGF from VEGFR-2 binding and modulates VEGFR-2 signaling. VEGF-A, the most dangerous type to human health, binds to both VEGFR-1 and VEGFR-2. VEGFR antagonists are mostly used, or under investigation, for treating cancers. Lenvima, acting as a multiple kinase inhibitor against VEGFR-1, VEGFR-2 and VEGFR-3 kinases, was approved in 2015 for the treatment of differentiated thyroid cancer, and in 2016 for treatment of advanced renal cell carcinoma in combination with Everolimus.

### Fc and FcR

The fragment crystallizable region (Fc region) is the tail region of an antibody and is the domain that determines the effector function of the antibody, that is, how it engages with specific cell receptors or other defense proteins.

An Fc receptor (FcR) is a protein expressed on the surfaces of certain cells, including B lymphocytes, follicular dendritic cells, natural killer cells, macrophages, neutrophils, eosinophils, basophils, and mast cells. These cells contribute to the protective functions of the immune system.

An Fc region may interact with Fc receptors and some proteins of the complement system, activating the immune system and the complement system.

### Therapeutic bi-specific or multi-specific fusion proteins/antibodies

The antibody that targets a single antigen may have limited efficacy. For example, the overall response rate is only 33% for Avelumab (BAVENCIO^{®}), an anti-PD-L1 antibody that has been approved.

Bi- or tri-specific fusion proteins have been recently developed, and these fusion proteins have shown good effects in the pre-clinical and clinical trials.

Although attaching additional binding moieties to conventional antibodies seems conceptually straightforward, such modification significantly alters antibody structures and may compromise one another's affinity and/or efficacy (Wang S et al., (2021) EMBO Mol Med. 13(9):e14291). In order to optimize *in vivo* efficacy and pharmaceutical properties, elaborate design and engineering should be given to choice of main and appended binding moieties (sequences), balanced affinities for targets, sites of attachment (N- or C- termini, of heavy or light chains), structural stability, linker lengths and/or sequences (Shim H. (2020) Biomolecules. 10(3):360*).*

Citation or identification of any document in this application is not an admission that such a document is available as prior art to the present disclosure.

### SUMMARY OF THE INVENTION

The present application provides a novel recombinant fusion protein comprising an anti-PD-L1 antibody and a VEGF binding peptide. Compared to anti-PD-L1 antibodies, and VEGF binding proteins (including anti-VEGF antibodies), the recombinant fusion protein of the disclosure is able to bind PD-L1⁺, VEGF⁺ and/or PD-L1⁺VEGF⁺ cells at comparable activity, to induce antibody dependent cell-mediated cytotoxicity (ADCC) and antibody dependent cellular phagocytosis (ADCP) at comparable level against PD-L1⁺VEGF⁺ cells, and/or to block PD-1-PD-L1 interaction and VEGF-VEGFR interaction at comparable activity. In addition, the recombinant fusion protein of the disclosure showed better anti-tumor effect in the *in vivo* test than the combined use of the VEGF binding protein and the anti-PD-L1 antibody.

Specifically, the present application discloses a recombinant fusion protein, which comprises 1) an anti-PD-L1 antibody that specifically binds PD-L1, or an antigen-binding fragment thereof, and 2) a VEGF binding peptide that specifically binds VEGF, wherein the VEGF binding peptide and the anti-PD-L1 antibody or the antigen-binding fragment thereof are linked together. The anti-PD-L1 antibody or the antigen-binding fragment thereof may comprise a heavy chain variable region and a light chain variable region, wherein the heavy chain variable region may comprise HV-CDR1, HV-CDR2 and HV-CDR3, wherein the HV-CDR1, HV-CDR2 and HV-CDR3 may comprise the amino acid sequences of SEQ ID NOs: 1, 2 and 3, respectively, wherein the light chain variable region may comprise LV-CDR1, LV-CDR2 and LV-CDR3, wherein the LV-CDR1, and LV-CDR3 may comprise the amino acid sequences of SEQ ID NOs: 4, and 5, respectively, the LV-CDR2 may comprise the amino acid sequence "YTS". The anti-PD-L1 antibody or the antigen-binding fragment thereof may comprise a heavy chain constant region, which may be linked to the C-terminus of the heavy chain variable region. The heavy chain constant region may comprise FcR binding capability. In certain embodiments, the anti-PD-L1 antibody or the antigen-binding fragment thereof may comprise a heavy chain variable region, a heavy chain constant region and a light chain variable region, wherein the heavy chain variable region may comprise HV-CDR1, HV-CDR2 and HV-CDR3, wherein the HV-CDR1, HV-CDR2 and HV-CDR3 may comprise the amino acid sequences of SEQ ID NOs: 1, 2 and 3, respectively, wherein the light chain variable region may comprise LV-CDR1, LV-CDR2 and LV-CDR3, wherein the LV-CDR1, and LV-CDR3 may comprise the amino acid sequences of SEQ ID NOs: 4, and 5, respectively, the LV-CDR2 may comprise the amino acid sequence "YTS", wherein the heavy chain constant region may comprise FcR binding ability and is linked to the C terminus of the heavy chain variable region. The VEGF binding peptide may be linked to the N-terminus of the heavy chain variable region or the light chain variable region of the anti-PD-L1 antibody or the antigen-binding fragment thereof.

The VEGF binding peptide may be an extracellular Ig-like domain of vascular endothelial growth factor receptor 1 (VEGFR1). The VEGF binding peptide may be a second extracellular Ig-like domain of VEGFR1 (VEGFR1D2). In certain embodiments, the VEGF binding peptide may comprise an amino acid sequence having at least 80%, 85%, 90%, 91%, 92%, 93%, 94%, 95%, 96%, 97%, 98%, 99% or 100% sequence identity to SEQ ID NO: 11.

The heavy chain variable region of the anti-PD-L1 antibody or the antigen-binding fragment thereof may comprise an amino acid sequence having at least 80%, 85%, 90%, 91%, 92%, 93%, 94%, 95%, 96%, 97%, 98%, or 99% sequence identity to SEQ ID NO: 7. In an embodiment, the heavy chain variable region may comprise the amino acid sequence of SEQ ID NO: 7. The light chain variable region of the anti-PD-L1 antibody or the antigen-binding fragment thereof may comprise an amino acid sequence having at least 80%, 85%, 90%, 91%, 92%, 93%, 94%, 95%, 96%, 97%, 98%, or 99% sequence identity to SEQ ID NO: 8. In an embodiment, the light chain variable region may comprise the amino acid sequence of SEQ ID NO: 8. In an embodiment, the heavy chain variable region and the light chain variable region of the anti-PD-L1 antibody or the antigen-binding fragment thereof may comprise amino acid sequences having at least 80%, 85%, 90%, 91%, 92%, 93%, 94%, 95%, 96%, 97%, 98%, or 99% sequence identity to SEQ ID NOs: 7 and 8, respectively. In certain embodiments, the heavy chain variable region and the light chain variable region of the anti-PD-L1 antibody or the antigen-binding fragment thereof may comprise the amino acid sequences of SEQ ID NOs: 7 and 8, respectively.

The heavy chain constant region may be a naturally occurring or engineered human IgG1, IgG2, IgG3 or IgG4 heavy chain constant region, or a functional fragment thereof. The heavy chain constant region having FcR binding ability may be a naturally occurring or engineered human IgG1, IgG2, IgG3 or IgG4 heavy chain constant region, or a functional fragment thereof. In certain embodiments, the heavy chain constant region having FcR binding ability may be a human IgG1 heavy chain constant region, or a functional fragment thereof. In certain embodiments, the heavy chain constant region having FcR binding ability may comprise an amino acid sequence having at least 80%, 85%, 90%, 91%, 92%, 93%, 94%, 95%, 96%, 97%, 98%, 99% or 100% sequence identity to SEQ ID NO: 9.

The anti-PD-L1 antibody or the antigen-binding fragment thereof may further comprise a light chain constant region, such as a human κ light chain constant region, or a functional fragment thereof, linked to the C terminus of the light chain variable region. In certain embodiments, the light chain constant region may comprise an amino acid sequence having at least 80%, 85%, 90%, 91%, 92%, 93%, 94%, 95%, 96%, 97%, 98%, 99% or 100% sequence identity to SEQ ID NO: 10.

The heavy chain of the anti-PD-L1 antibody or the antigen-binding fragment thereof may comprise a heavy chain variable region and a heavy chain constant region. The heavy chain may comprise an amino acid sequence having at least 80%, 85%, 90%, 91%, 92%, 93%, 94%, 95%, 96%, 97%, 98%, or 99% sequence identity to SEQ ID NO: 15. In an embodiment, the heavy chain may comprise the amino acid sequence of SEQ ID NO: 15. The light chain of the anti-PD-L1 antibody or the antigen-binding fragment thereof may comprise a light chain variable region and a light chain constant region. The light chain may comprise an amino acid sequence having at least 80%, 85%, 90%, 91%, 92%, 93%, 94%, 95%, 96%, 97%, 98%, or 99% sequence identity to SEQ ID NO: 14. In an embodiment, the light chain may comprise the amino acid sequence of SEQ ID NO: 14. In an embodiment, the heavy chain and the light chain of the anti-PD-L1 antibody or the antigen-binding fragment thereof may comprise amino acid sequences having at least 80%, 85%, 90%, 91%, 92%, 93%, 94%, 95%, 96%, 97%, 98%, or 99% sequence identity to SEQ ID NOs: 15 and 14, respectively. In certain embodiments, the heavy chain and the light chain of the anti-PD-L1 antibody or the antigen-binding fragment thereof may comprise the amino acid sequences of SEQ ID NOs: 15 and 14, respectively.

At least one paratope of the anti-PD-L1 antibody or antigen-binding fragment thereof may be linked to the VEGF binding peptide at the N-terminus of a heavy chain variable region or a light chain variable region constituting that paratope. In certain embodiments, each paratope of the anti-PD-L1 antibody or antigen-binding fragment thereof may be linked to the VEGF binding peptide at the N-terminus of a heavy chain variable region or a light chain variable region constituting that paratope. In certain embodiments, each paratope of the anti-PD-L1 antibody or antigen-binding fragment thereof may be linked to the VEGF binding peptide at the N-terminus of a heavy chain variable region constituting that paratope. In certain embodiments, each paratope of the anti-PD-L1 antibody or antigen-binding fragment thereof may be linked to the VEGF binding peptide at the N-terminus of a light chain variable region constituting that paratope.

The anti-PD-L1 antibody or antigen-binding fragment thereof of the disclosure may be linked via a linker to the VEGF binding peptide. The linker may be a peptide of 5 to 30, 10 to 30, 10 to 20, or 15 amino acid residues. The linker may be a GS linker, e.g., -(Gly-Gly-Gly-Gly-Ser)₃- (SEQ ID NO: 12).

The recombinant fusion protein of the disclosure may comprise a chain of VEGF binding peptide-linker-anti-PD-L1 heavy chain, and an anti-PD-L1 light chain, wherein the chain of VEGF binding peptide-linker-anti-PD-L1 heavy chain may comprise an amino acid sequence having at least 80%, 85%, 90%, 91%, 92%, 93%, 94%, 95%, 96%, 97%, 98%, or 99% sequence identity to SEQ ID NO: 13, and the anti-PD-L1 light chain may comprise an amino acid sequence having at least 80%, 85%, 90%, 91%, 92%, 93%, 94%, 95%, 96%, 97%, 98%, or 99% sequence identity to SEQ ID NO: 14. In certain embodiments, the recombinant fusion protein of the disclosure may comprise a chain of VEGF binding peptide-linker-anti-PD-L1 heavy chain, and an anti-PD-L1 light chain, wherein the chain of VEGF binding peptide-linker-anti-PD-L1 heavy chain may comprise the amino acid sequence of SEQ ID NO: 13, and the anti-PD-L1 light chain may comprise the amino acid sequence of SEQ ID NO: 14. The amino acid sequences of SEQ ID NOs: 13 and 14 may be encoded by the nucleotide sequences of SEQ ID NOs: 23 and 24, respectively.

In an embodiment, the recombinant fusion protein of the disclosure comprises:
i) a first polypeptide chain, comprising, from N-terminus to C-terminus, a VEGF binding peptide, a linker, an anti-PD-L1 heavy chain variable region, and a heavy chain constant region;
ii) a second polypeptide chain, comprising, from N-terminus to C-terminus, an anti-PD-L1 light chain variable region, and a light chain constant region;
iii) a third polypeptide chain, comprising, from N-terminus to C-terminus, a VEGF binding peptide, a linker, an anti-PD-L1 heavy chain variable region, and a heavy chain constant region; and
iv) a fourth polypeptide chain, comprising, from N-terminus to C-terminus, an anti-PD-L1 light chain variable region, and a light chain constant region,
wherein the anti-PD-L1 heavy chain variable region in the first polypeptide chain and the anti-PD-L1 light chain variable region in the second polypeptide chain are able to associate and form an antigen binding domain specifically against PD-L1, the anti-PD-L1 heavy chain variable region in the third polypeptide chain and the anti-PD-L1 light chain variable region in the fourth polypeptide chain are able to associate and form an antigen binding domain specifically against PD-L1, and the heavy chain constant region in the first polypeptide chain and the heavy chain constant region in the third polypeptide chain are able to be associated together via e.g., the knobs-into-holes approach, the covalent bond(s) and/or the disulfide bond(s).

The recombinant fusion protein of the disclosure may comprise an anti-PD-L1 heavy chain, and a chain of VEGF binding peptide-linker-anti-PD-L1 light chain, wherein the anti-PD-L1 heavy chain may comprise an amino acid sequence having at least 80%, 85%, 90%, 91%, 92%, 93%, 94%, 95%, 96%, 97%, 98%, or 99% sequence identity to SEQ ID NO: 15, and the chain of VEGF binding peptide-linker-anti-PD-L1 light chain may comprise an amino acid sequence having at least 80%, 85%, 90%, 91%, 92%, 93%, 94%, 95%, 96%, 97%, 98%, or 99% sequence identity to SEQ ID NO: 16. In certain embodiments, the recombinant fusion protein of the disclosure may comprise an anti-PD-L1 heavy chain, and a chain of VEGF binding peptide-linker-anti-PD-L1 light chain, wherein the anti-PD-L1 heavy chain may comprise the amino acid sequence of SEQ ID NO: 15, and the chain of VEGF binding peptide-linker- anti-PD-L1 light chain may comprise the amino acid sequence of SEQ ID NO: 16. The amino acid sequences of SEQ ID NOs: 15 and 16 may be encoded by the nucleotide sequences of SEQ ID NOs: 25 and 26, respectively.

In an embodiment, the recombinant fusion protein of the disclosure comprises:
i) a first polypeptide chain, comprising, from N-terminus to C-terminus, an anti-PD-L1 heavy chain variable region, and a heavy chain constant region;
ii) a second polypeptide chain, comprising, from N-terminus to C-terminus, a VEGF binding peptide, a linker, an anti-PD-L1 light chain variable region, and a light chain constant region;
iii) a third polypeptide chain, comprising, from N-terminus to C-terminus, an anti-PD-L1 heavy chain variable region, and a heavy chain constant region; and
iv) a fourth polypeptide chain, comprising, from N-terminus to C-terminus, a VEGF binding peptide, a linker, an anti-PD-L1 light chain variable region, and a light chain constant region,
wherein the anti-PD-L1 heavy chain variable region in the first polypeptide chain and the anti-PD-L1 light chain variable region in the second polypeptide chain are able to associate and form an antigen binding domain specifically against PD-L1, the anti-PD-L1 heavy chain variable region in the third polypeptide chain and the anti-PD-L1 light chain variable region in the fourth polypeptide chain are able to associate and form an antigen binding domain specifically against PD-L1, and the heavy chain constant region in the first polypeptide chain and the heavy chain constant region in the third polypeptide chain are able to be associated together via e.g., the knobs-into-holes approach, the covalent bond(s) and/or the disulfide bond(s).

The present application also relates to an anti-PD-L1 antibody or an antigen-binding fragment thereof of the disclosure, which may comprise a heavy chain variable region and a light chain variable region, wherein the heavy chain variable region may comprise HV-CDR1, HV-CDR2 and HV-CDR3, wherein the HV-CDR1, HV-CDR2 and HV-CDR3 may comprise the amino acid sequences of SEQ ID NOs: 1, 2 and 3, respectively, wherein the light chain variable region may comprise LV-CDR1, LV-CDR2 and LV-CDR3, wherein the LV-CDR1, and LV-CDR3 may comprise the amino acid sequences of SEQ ID NOs: 4, and 5, respectively, the LV-CDR2 may comprise the amino acid sequence "YTS". The heavy chain variable region may comprise an amino acid sequence having at least 80%, 85%, 90%, 91%, 92%, 93%, 94%, 95%, 96%, 97%, 98%, 99% or 100% sequence identity to SEQ ID NO: 7. The light chain variable region may comprise an amino acid sequence having at least 80%, 85%, 90%, 91%, 92%, 93%, 94%, 95%, 96%, 97%, 98%, 99% or 100% sequence identity to SEQ ID NO: 8. The anti-PD-L1 antibody or antigen-binding fragment thereof may further comprise a heavy chain constant region and/or a light chain constant region. The heavy chain constant region may be a human IgG1, IgG2, IgG3 or IgG4 heavy chain constant region, or a functional fragment thereof, linked to the C-terminus of the heavy chain variable region. In certain embodiments, the heavy chain constant region may be one with FcR binding ability, or a functional fragment thereof. In certain embodiments, the heavy chain constant region may be human IgG1 heavy chain constant region, or a functional fragment thereof. In certain embodiments, the heavy chain constant region may comprise the amino acid sequence of SEQ ID NO: 9. The light chain constant region may be a human κ constant region, or a functional fragment thereof, linked to the C-terminus of the light chain variable region. In certain embodiments, the light chain constant region may comprise the amino acid sequence of SEQ ID NO: 10. The anti-PD-L1 antibody or the antigen-binding fragment thereof of the disclosure, as compared to the prior art anti-PD-L1 antibodies such as Atezolizumab, comprises comparable PD-L1 binding activity/affinity, and comparable PD-1-PD-L1 blocking activity. The anti-PD-L1 antibody or the antigen-binding fragment thereof of the disclosure may be able to induce ADCC and ADCP against PD-L1⁺ cells.

The disclosure further provides a nucleic acid molecule encoding the recombinant fusion protein, the anti-PD-L1 antibody or the antigen-binding fragment thereof of the disclosure, as well as an expression vector that comprises the nucleic acid molecule and a host cell that comprises the expression vector or comprises the nucleic acid molecule integrated in its genome. A method for preparing the recombinant fusion protein, the anti-PD-L1 antibody or the antigen-binding fragment thereof of the disclosure using the host cell of the disclosure is also provided, that comprises (i) expressing the recombinant fusion protein, the anti-PD-L1 antibody or the antigen-binding fragment thereof in the host cell and (ii) isolating the recombinant fusion protein, the anti-PD-L1 antibody or the antigen-binding fragment thereof from the host cell or its cell culture.

The disclosure further provides a pharmaceutical composition that may comprise the recombinant fusion protein, the anti-PD-L1 antibody or the antigen-binding fragment thereof, the nucleic acid molecule, the expression vector, or the host cell of the disclosure, and a pharmaceutically acceptable carrier. In certain embodiments, the pharmaceutical composition comprises at least one pharmaceutically acceptable adjuvant.

The pharmaceutical composition of the disclosure may be used to treat a disease associated with VEGF and/or PD-L1 over-expression/signaling, or to prepare a medicament for treating a disease associated with VEGF and/or PD-L1 over-expression/signaling.

In one aspect, the disclosure provides a method for treating or alleviating a disease associated with VEGF and/or PD-L1 signaling/over-expression in a subject in need thereof, comprising administering to the subject a therapeutically acceptable amount of the pharmaceutical composition of the disclosure.

The pharmaceutical composition of the disclosure may be administered via the intraperitoneal, subcutaneous, or intravenous route. In one embodiment, the pharmaceutical composition of the disclosure may be administered by intraperitoneal injection, subcutaneous injection, or intravenous injection.

The disease associated with VEGF and/or PD-L1 signaling/over-expression may be cancer, including the solid cancer and the hematological cancer. The cancer may be acute myelocytic leukemia (AML), chronic myelocytic leukemia (CML), acute lymphoblastic leukemia (ALL), non-Hodgkin's lymphoma (NHL), multiple myeloma (MM), bladder cancer, ovarian cancer, prostate cancer, lung cancer, colon cancer, breast cancer, pancreatic cancer, liver cancer, renal cell carcinoma, melanoma, glioblastomas, cervical cancer or angiosarcoma.

The disease associated with VEGF and/or PD-L1 signaling/over-expression may be age-related macular degeneration (AMD), diabetic retinopathy, or liver fibrosis.

Other features and advantages of the instant disclosure will be apparent from the following detailed description and examples which should not be construed as limiting. The contents of all references, GenBank entries, patents and published patent applications cited throughout this application are expressly incorporated herein by reference.

### DESCRIPTION OF THE DRAWINGS

The following detailed description, given by way of example, but not intended to limit the application solely to the specific embodiments described, may best be understood in conjunction with the accompanying drawings.

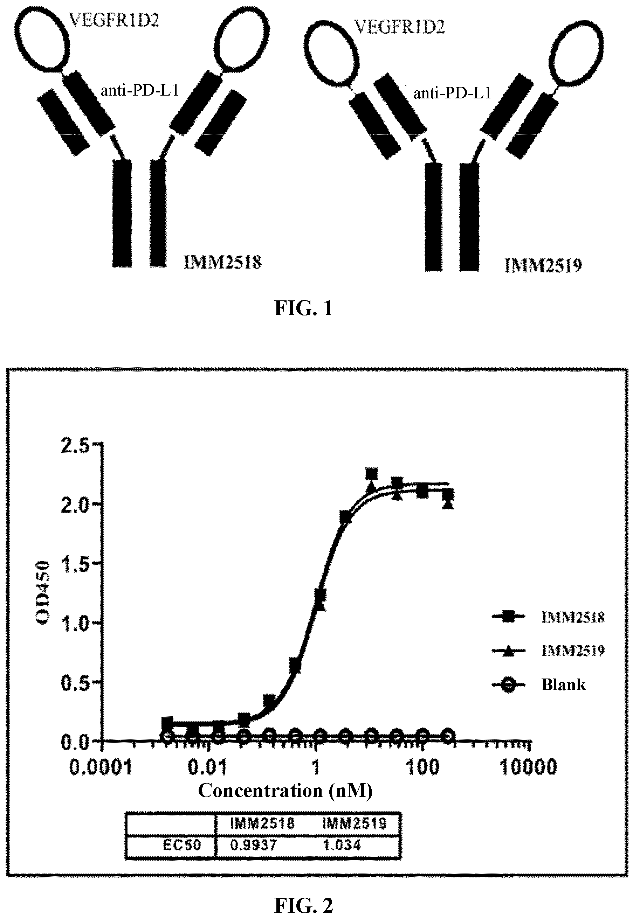

FIG. 1 is a schematic diagram of the structures of the recombinant fusion proteins IMM2518 and IMM2519 of the disclosure. The circular components on the top represent VEGFR1D2, which are each linked via a linker to the N-terminus of the heavy chain (left panel, IMM2518) or the light chain (right panel, IMM2519) of the anti-PD-L1 antibody IMM2515H. The VEGFR1D2 comprises the amino acid sequence of SEQ ID NO: 11. The linker comprises the amino acid sequence of SEQ ID NO: 12. The heavy chain of IMM2515H comprises the amino acid sequence of SEQ ID NO: 15, and the light chain comprises the amino acid sequence of SEQ ID NO: 14.
FIG. 2 shows the binding activity of IMM2518 and IMM2519 to VEGF.
FIG. 3 shows the binding activity of IMM2518 and IMM2519 to PD-L1⁺ CHO cells, with IMM2515H used as the positive control, and hIgG1-Fc used as the negative control.
FIG. 4 shows the binding activity of IMM2518 to PD-L1⁺ CHO cells, with IMM2510, IMM2515H and atezolizumab used as the positive controls, and hIgG1-Fc used as the negative control.
FIG. 5 shows the blocking activity of IMM2518 on PD-1 binding to PD-L1⁺CHO cells, with IMM2510, IMM2515H and atezolizumab used as the positive controls, and hIgG1-Fc used as the negative control.
FIG. 6 shows the blocking activity of IMM2518 on VEGF binding to VEGFR⁺ Jurkat cells, with IMM2510, VEGFR1-Fc and bevacizumab used as the positive controls, and hIgG1 used as the negative control.
FIG. 7 shows the binding activity of IMM2518 to human PD-L1, rat PD-L1, mouse PD-L1, monkey PD-L1 and human PD-L2.
FIGs. 8A to 8C show the activity of IMM2518 to trigger ADCC against PD-L1⁺ Raji cells (A), RKO cells (B) and HCC827 cells (C), with IMM2515H, IMM2510 and atezolizumab used as the positive controls, and hIgG1-Fc used as the negative control.
FIG. 9 shows the activity of IMM2518 to induce ADCP against RKO cells, with IMM2510, IMM2515H and atezolizumab used as the positive controls, and hIgG1-Fc used as the negative control.
FIG. 10 shows the tumor size change in tumor-bearing mice administered with IMM2518, with IMM2510, IMM2515H, VEGFR1D2-Fc, and the IMM2515H+VEGFR1D2-Fc combination used as the controls.
FIG. 11 shows the tumor size change in tumor-bearing mice administered with IMM2518 via intraperitoneal injection, subcutaneous injection, or intravenous injection.

### DETAILED DESCRIPTION OF THE APPLICATION

There are principally three different approaches to targeting two or more pharmacologies of tumor growth. Most commonly, patients can be given a cocktail of two or more different drugs. Although this option allows for maximal flexibility with respect to possible drug combinations and different dosages, it suffers from a) potentially poor adherence to treatment by the patient because of the increased pill burden and the different dosing schedules for the individual drugs; b) possible incompatibilities because of drug-drug interactions; and c) increased risk of drug side effects. These problems may reduce the effectiveness of therapy and hamper the attainment of treatment goals particularly in the management of chronic diseases such as cancer.

The second approach relies on the use of fixed-dose combinations of drugs in a single dosage form. This approach reduces pill burden, resulting in improved patient compliance. The disadvantage of fixed-dose combinations is primarily the limited choice of possible dose ratios between the active ingredients, which makes it more difficult to properly titrate the individual patient to maximum efficacy with minimal adverse effects. In addition, different pharmacokinetic properties of the components in the combination might lead to a complex temporal mismatch in pharmacodynamic effects at the individual targets thereby compromising overall efficacy.

The third approach is the use of multifunctional drugs that combine two or more pharmacologies in a single entity. The design and validation of such multifunctional entities are more complex and require substantial investigation into the optimal ratio of target activities, and the combined drug pharmacokinetics may produce matched drug pharmacokinetics at the molecule targets level. Multifunctional molecules may also be amenable to fixed dose combination with other drugs thereby combining three or even four pharmacologies in a single pill to produce further increments in efficacy.

Through diligent experimentation, the inventors of the disclosure invented a novel recombinant multi-functional fusion protein, which can attach the tumor via three mechanisms of action. The first one is to release the check or inhibition on immune cells such as T cells by PD-1-mediated inhibitory signals, the second is to prohibit new blood vessel formation in the tumor microenvironment and release immunity suppression and thus limit target cell growth, and the other is to stimulate killing of target cells such as PD-L1⁺ tumor cells by immune cells such as NK cells and macrophages.

The recombinant fusion protein of the disclosure comprises an anti-PD-L1 antibody or an antigen-binding fragment thereof, wherein at least one paratope of the anti-PD-L1 antibody or antigen-binding fragment thereof is linked via a linker to an VEGF binding peptide at the N-terminus of the heavy chain or the light chain constituting that paratope. The recombinant fusion protein may simultaneously bind VEGF, PD-L1 and FcR, i) to block the interaction between the PD-L1 on cancer/tumor cell surfaces with the PD-L1 on immune cell surfaces (e.g., T cell surfaces), so as to release the check of the PD-1 mediated inhibitory signals on T cells, ii) to bind the FcR on immune cells such as NK cells and macrophages, to activate killing of target cells such as PD-L1⁺ tumor cells by NK cells or macrophages, and iii) to bind VEGF in e.g., the immune microenvironment, to reduce tumor angiogenesis and thus reduce blood and nutrient supply to tumor cells.

In one embodiment, at least one paratope of the anti-PD-L1 antibody or antigen-binding fragment thereof may be linked via a linker to the VEGF binding peptide at the N-terminus of a heavy chain or a light chain constituting that paratope. In another embodiment, each paratope of the anti-PD-L1 antibody or antigen-binding fragment thereof may be linked via a linker to the VEGF binding peptide at the N-terminus of a heavy chain or a light chain constituting that paratope. In an embodiment, each paratope of the anti-PD-L1 antibody or antigen-binding fragment thereof may be linked via a linker to the VEGF binding peptide at the N-terminus of a heavy chain constituting that paratope. In an embodiment, each paratope of the anti-PD-L1 antibody or antigen-binding fragment thereof may be linked via a linker to the VEGF binding peptide at the N-terminus of a light chain constituting that paratope. The recombinant fusion protein of the disclosure is small in size (15-180 kDa), with a relatively long half-life of 5 to 10 days.

The three main components contained in the recombinant fusion protein of the present disclosure are an VEGF binding peptide, a linker, and an anti-PD-L1 antibody or an antigen-binding fragment thereof. A person of ordinary skills in the art will recognize that there are many design choices for selecting the above three components. Preferably, human-derived sequence is used in human cancer therapies, as the strong immunogenicity of the proteins or peptides from non-human animals may lead to allergy and other adverse effects. However, other animal proteins or peptides, humanized if needed, may also be used in the present disclosure based on different application purposes.

Any extracellular Ig-like domain of any VEGFR (VEGFR1, VEGFR2, and VEGFR3) capable of binding VEGF, especially VEGF-A, may be selected for construction of the recombinant protein of the disclosure. In an embodiment, the VEGFR in the recombinant fusion protein is VEGFR1, especially a second extracellular Ig-like domain of VEGFR1 (VEGFR1D2).

The VEGFR1D2 may comprise an amino acid sequence having at least 80%, 85%, 90%, 91%, 92%, 93%, 94%, 95%, 96%, 97%, 98% or 99% sequence identity to SEQ ID NO: 11, wherein the VEGFR1D2 can bind to VEGF molecules expressed by or around target cells, e.g., cancer/tumor cells, and thus limit growth of target cells. In an embodiment, VEGFR1D2 may comprise the amino acid sequence of SEQ ID NO: 11.

Linkers serve primarily as a spacer between the VEGF binding peptide and the N-terminus of the heavy chain or light chain of the anti-PD-L1 antibody. The linker may be made up of amino acids linked together by peptide bonds, preferably 5 to 30, 10 to 30, 10 to 20 or 15 amino acids linked by peptide bonds, wherein the amino acids are selected from the 20 naturally occurring amino acids. One or more of these amino acids may be glycosylated or aglycosylated, as is understood by those of skill in the art. In one embodiment, the 5 to 30, 10 to 30, 10 to 20 or 15 amino acids may be selected from glycine, alanine, proline, asparagine, glutamine, serine and lysine. In an embodiment, the linker is made up of a majority of amino acids that are sterically unhindered, such as glycine and alanine. Exemplary linkers are polyglycines (particularly Glys, poly(Gly-Ala)), and polyalanines. One exemplary suitable linker as shown in the Examples below is a GS linker, such as -(Gly-Gly-Gly-Gly-Ser)₃- (SEQ ID NO: 12).

Linkers may also be non-peptide linkers. For example, alkyl linkers such as -NH-, - (CH₂)s-C(O)-, wherein s=2-20, can be used. These alkyl linkers may further be substituted by any non-sterically hindering group such as lower alkyl (e.g., C₁₋₄ lower acyl), halogen (e.g., CI, Br), CN, NH2, phenyl, or the like.

In certain embodiments, the anti-PD-L1 antibody may be an isolated monoclonal antibody, and comprises two heavy chains and two light chains, each heavy chain comprising the amino acid sequence of SEQ ID NO: 15, each light chain comprising the amino acid sequence of SEQ ID NO: 14. The Fab portion (or the paratope) of the anti-PD-L1 antibody may bind to the PD-L1 on the surface of target cells such as cancer/tumor cells, blocking the interaction between the PD-L1 and the PD-1 on the surface of immune cells such as T cells, and thus releasing the check of the PD-1 mediated inhibitory signals on immune cells, while the Fc portion of the anti-PD-L1 antibody may bind to the FcR on immune cells such as NK cells and macrophages, triggering targeted cell killing by NK cells or macrophages. In certain embodiments, the heavy chain may comprise an amino acid sequence having at least 80%, 85%, 90%, 91%, 92%, 93%, 94%, 95%, 96%, 97%, 98% or 99% sequence identity to SEQ ID NO: 15, wherein the anti-PD-L1 antibody is able to bind PD-L1 and block PD-1-PD-L1 interaction, and to bind FcRs on NK cells or macrophages to trigger killing of cancer cells by NK cells and/or macrophages. In certain embodiments, the light chain may comprise an amino acid sequence having at least 80%, 85%, 90%, 91%, 92%, 93%, 94%, 95%, 96%, 97%, 98% or 99% sequence identity to SEQ ID NO: 14, wherein the anti-PD-L1 antibody is able to bind PD-L1 and block PD-1-PD-L1 interaction.

The term "antibody" herein includes IgG, IgA, IgD, IgE and IgM whole antibodies and any antigen-binding fragment (i.e., "antigen-binding portion") or single chains thereof. Whole antibodies are glycoproteins comprising at least two heavy (H) chains and two light (L) chains inter-connected by disulfide bonds. Each heavy chain comprises a heavy chain variable region (V_{H}) and a heavy chain constant region. The heavy chain constant region comprises three domains, C_{H1}, C_{H2} and C_{H3}. Each light chain comprises a light chain variable region (V_{L}) and a light chain constant region. The light chain constant region comprises one domain, C_{L}. The V_{H} and V_{L} regions can be further subdivided into regions of hypervariability, termed CDR regions, interspersed with framework regions (FR) that are more conserved. Each V_{H} and V_{L} is composed of three CDRs and four FRs, arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies can mediate the binding of the immunoglobulins to host tissues or factors, including various cells of the immune system (e.g., effector cells) and the first component (C1q) of the classical complement system. The "functional fragment" of a heavy chain constant region refers to the part of the heavy chain constant region that retains the binding affinity to multiple immune system cells (e.g., effector cells) and the first component (C1q) of the complement system, such as the Fc region, the hinge-Fc region, and the like.

The "antigen-binding fragment" or "antigen-binding portion" of an antibody refers to one or more fragments of an antibody that retain the ability to specifically bind to an antigen (e.g., a PD-L1 protein). It has been shown that the antigen-binding function of an antibody can be performed by fragments of a full-length antibody. Examples of binding fragments encompassed within the term "antigen-binding fragment" of an antibody include (i) a Fab fragment, a monovalent fragment consisting of the V_{L}, V_{H}, C_{L} and C _{H1} domains; (ii) a F(ab')₂ fragment, a bivalent fragment which may comprise two Fab fragments linked by a disulfide bridge at the hinge region; (iii) a Fd fragment consisting of the V_{H} and C_{H1} domains; (iv) a Fv fragment consisting of the V_{L} and V_{H} domains of a single arm of an antibody, (v) a dAb fragment, which consists of a V_{H} domain (Ward et al., (1989) Nature 341:544-546); (vi) an isolated complementarity determining region (CDR); and (viii) a fragment containing a heavy chain variable region, a light chain variable region, and a Fc region or a hinge-Fc region. Furthermore, although the two domains of the Fv fragment, V_{L} and V_{H}, are coded by separate genes, they can be joined, using recombinant methods, by a synthetic linker that enables them to be made as a single protein chain in which the V_{L} and V_{H} regions pair to form monovalent molecules (known as single chain Fv (scFv). Such single chain antibodies are also intended to be encompassed within the term. These antigen binding fragments are obtained using conventional techniques known to those with skill in the art, and the fragments may be screened for utility in the same manner as are intact antibodies.

The heavy chain variable region CDRs and the light chain variable region CDRs of the antibody or antigen-binding fragment of the disclosure have been defined by the IMGT numbering system. As is well known in the art, the heavy and light chain variable region CDRs can also be determined by e.g., Chothia, Kabat, AbM, or Contact numbering system/method.

The term "antibody dependent cytotoxicity", "antibody dependent cell mediated cytotoxicity" or "ADCC" refers to a cell mediated immune defense where the immune effector cells actively lyse target cells such as cancer cells whose cell surface antigens are bound by the antibodies (e.g., anti-PD-L1 antibodies) or VEGF binding peptides.

The term "antibody dependent cellular phagocytosis" or "ADCP" refers to the antibody mediated phagocytosis of target cells by macrophages or neutrophils where the antibody binds to the antigen epitopes on the target cells via its Fab portion and binds to the FcRs of the macrophages or neutrophils via its Fc portion.

The term "subject" includes any human or nonhuman animal. The term "nonhuman animal" includes all vertebrates, e.g., mammals and non-mammals, such as non-human primates, sheep, dogs, cats, cows, horses, chickens, amphibians, and reptiles, although mammals are preferred, such as non-human primates, sheep, dogs, cats, cows and horses.

The term "EC₅₀", also known as half maximal effective concentration, refers to the concentration of a molecule (e.g., an antibody, a fusion protein, and the like) that produces 50% of the maximal effect.

The term "IC₅₀", also known as half maximal inhibitory concentration, refers to the concentration of an inhibitor (e.g., an antibody, a fusion protein, and the like) which inhibits a specific biological process by half.

The term "therapeutically effective amount" means an amount of a molecule (e.g., an antibody, a fusion protein, or the like) sufficient to prevent or ameliorate the symptoms associated with a disease or condition (such as a cancer). A therapeutically effective amount is understood to be in context to the condition being treated, where the actual effective amount is readily discerned by those of skill in the art.

As used herein, "sequence identity" refers to the percent of nucleotides/amino acid residues in a subject sequence that are identical to nucleotides/amino acid residues in a reference sequence, after aligning the sequences and, if necessary, introducing gaps, to achieve the maximum percent sequence identity between the sequences. Pairwise and multiple sequence alignment for the purposes of determining percent sequence identity between two or more amino acid or nucleic acid sequences can be achieved in various ways known to a person of skill in the art, for instance, using the publicly available computer software such as ClustalOmega, T-coffee, Kalign and MAFFT.

Also, the present disclosure provides a polynucleotide encoding the recombinant fusion protein, the anti-PD-L1 antibody or the antigen-binding portion thereof, and an expression vector expressing the recombinant fusion protein. Examples of vectors include but are not limited to plasmids, viral vectors, yeast artificial chromosomes (YACs), bacterial artificial chromosomes (BACs), transformation-competent artificial chromosomes (TACs), mammalian artificial chromosomes (MACs) and human artificial episomal chromosomes (HAECs).

The present disclosure provides host cells comprising the above expression vectors. The host cells may be transformed or transfected with the expression vectors. Suitable host cells include *Escherichia coli* (*E. coli*), yeasts and other eukaryotes. Preferably, *Escherichia coli,* yeast or mammalian cell lines (such as COS or CHO) are used.

In another aspect, the present disclosure provides a pharmaceutical composition comprising the recombinant fusion protein, the anti-PD-L1 antibody or the antigen-binding fragment thereof, the nucleic acid molecule, the expression vector or the host cell of the present disclosure formulated together with a pharmaceutically acceptable carrier. The pharmaceutical composition may comprise an excipient, and/or an adjuvant. The composition may optionally contain one or more additional pharmaceutically active ingredients, such as another antibody or another drug. The pharmaceutical composition of the disclosure can also be administered in a combination therapy with, for example, another immune-stimulatory agent, anti-cancer agent, an anti-viral agent, or a vaccine.

The pharmaceutical composition can comprise any number of excipients. Excipients that can be used include carriers, surface active agents, thickening or emulsifying agents, solid binders, dispersion or suspension aids, solubilizers, colorants, flavoring agents, coatings, disintegrating agents, lubricants, sweeteners, preservatives, isotonic agents, and combinations thereof. The selection and use of suitable excipients are taught in Gennaro, ed., Remington: The Science and Practice of Pharmacy, 20th Ed. (Lippincott Williams & Wilkins 2003), the disclosure of which is incorporated herein by reference.

The primary vehicle or carrier in a pharmaceutical composition may be either aqueous or non-aqueous in nature. For example, a suitable vehicle or carrier may be water for injection, physiological saline solution or artificial cerebrospinal fluid, possibly supplemented with other materials common in injection. For example, the vehicle or carrier may be neutral buffered saline or saline mixed with serum albumin. Other exemplary pharmaceutical compositions comprise Tris buffers, or acetate buffers, which may further include sorbitol or a suitable substitute thereof. In one embodiment of the present disclosure, compositions may be prepared for storage by mixing the selected composition having the desired degree of purity with optional formulation agents (Remington's Pharmaceutical Sciences, supra) in the form of a lyophilized cake or an aqueous solution. Further, the therapeutic composition may be formulated as a lyophilizate using appropriate excipients such as sucrose.

The pharmaceutical composition may be used for intravenous, intramuscular, subcutaneous, parenteral, spinal or epidermal administration (e.g., by injection or infusion). Depending on the route of administration, the active molecule can be coated in a material to protect it from the action of acids or enzymes and other natural conditions that may inactivate it. The phrase "parenteral administration" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion. Alternatively, an antibody of the disclosure can be administered via a non-parenteral route, such as a topical, epidermal or mucosal route of administration, e.g., intranasally, orally, vaginally, rectally, sublingually or topically. In certain embodiments, the pharmaceutical composition may be administered via the intraperitoneal, subcutaneous, or intravenous route.

Pharmaceutical compositions can be in the form of sterile aqueous solutions or dispersions. They can also be formulated in a microemulsion, liposome, or other ordered structure suitable to high drug concentration.

The amount of active ingredient which can be combined with a carrier material to produce a single dosage form will vary depending upon the subject being treated and the particular mode of administration and will generally be that amount of the composition which produces a therapeutic effect. Generally, out of one hundred percent, this amount will range from about 0.01% to about 99% of active ingredient in combination with a pharmaceutically acceptable carrier.

Dosage regimens are adjusted to provide the optimum desired response (e.g., a therapeutic response). For example, several divided doses can be administered over time or the dose can be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the subjects to be treated; each unit contains a predetermined quantity of active molecule calculated to produce the desired therapeutic effect in association with the required pharmaceutical carrier. Alternatively, the recombinant protein can be administered as a sustained release formulation, in which case less frequent administration is required.

For administration of the fusion protein, the dosage may range from about 0.0001 to 100 mg/kg of the recipient body weight. An exemplary treatment regime entails administration twice per week.

A "therapeutically effective dosage" of a fusion protein of the disclosure preferably results in a decrease in severity of disease symptoms, an increase in frequency and duration of disease symptom-free periods, or a prevention of impairment or disability due to the disease affliction. For example, for the treatment of tumor-bearing subjects, a "therapeutically effective dosage" preferably inhibits tumor growth by at least about 40%, more preferably by at least about 60%, more preferably by at least about 80%, and more preferably by at least about 99%, relative to untreated subjects. A therapeutically effective amount of a fusion protein of the present disclosure can decrease tumor size, or otherwise ameliorate symptoms in a subject, which is typically a human or can be another mammal.

The pharmaceutical composition can be a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. See, e.g., Sustained and Controlled Release Drug Delivery Systems, J. R. Robinson, ed., Marcel Dekker, Inc., New York, 1978.

Pharmaceuticcal compositions can be administered via medical devices such as (1) needleless hypodermic injection devices (e.g., U.S. Pat. Nos. 5,399,163; 5,383,851; 5,312,335; 5,064,413; 4,941,880; 4,790,824; and 4,596,556); (2) micro-infusion pumps (U.S. Pat. No. 4,487,603); (3) transdermal devices (U.S. Pat.No. 4,486,194); (4) infusion apparatuses (U.S. Pat.Nos. 4,447,233 and 4,447,224); and (5) osmotic devices (U.S. Pat. Nos. 4,439,196 and 4,475,196); the disclosures of which are incorporated herein by reference.

In certain embodiments, the fusion protein of the disclosure can be formulated to ensure proper distribution *in vivo.* For example, to ensure that the therapeutic fusion protein of the disclosure cross the blood-brain barrier, the fusion protein can be formulated in liposomes, which may additionally comprise targeting moieties to enhance selective transport to specific cells or organs. See, e.g. U.S. Pat. Nos. 4,522,811; 5,374,548; 5,416,016; and 5,399,331.

A gene therapy *in vivo* is also envisioned wherein a nucleic acid molecule encoding the fusion protein of the present disclosure, or a derivative thereof is introduced directly into the subject. For example, a nucleic acid sequence encoding a recombinant fusion protein of the present disclosure is introduced into target cells via local injection of a nucleic acid construct with or without an appropriate delivery vector, such as an adeno-associated virus vector. Alternative viral vectors include, but are not limited to, retroviruses, adenovirus, herpes simplex vims and papilloma virus vectors. Physical transfer of the virus vector may be achieved *in vivo* by local injection of the desired nucleic acid construct or other appropriate delivery vector containing the desired nucleic acid sequence, liposome- mediated transfer, direct injection (naked DNA), or microparticle bombardment (gene-gun).

The composition of the present disclosure may be used alone or in combination with other therapeutic agents to enhance the therapeutic effects or decrease potential side effects.

Another object of the present disclosure is to provide a method for preparing the recombinant fusion protein, the anti-PD-L1 antibody or the antigen-binding fragment thereof described above. In one embodiment, the method comprises the steps of (1) providing a nucleic acid molecule encoding a recombinant fusion protein, an anti-PD-L1 antibody or its antigen-binding fragment thereof; (2) constructing an expression vector comprising the nucleic molecule of (1); (3) transfecting or transforming suitable host cells with the expression vector of (2) and cultivating the host cells to express the recombinant fusion protein, the anti-PD-L1 antibody or the antigen-binding fragment thereof; and (4) purifying the recombinant fusion protein, the anti-PD-L1 antibody or the antigen-binding fragment thereof. The preparation may be carried out with well-known technologies by an ordinarily skilled artisan.

Another object of the present disclosure is to provide a method of treating cancer using the pharmaceutical composition of the present disclosure, comprising administrating an effective amount of the aforementioned pharmaceutical composition to the patients or subjects in need thereof. In an embodiment, the pharmaceutical composition is used to treat cancers associated with VEGF and/or PD-L1 signaling/over-expression, including but not limited to, acute myelocytic leukemia (AML), chronic myelocytic leukemia (CML), acute lymphoblastic leukemia (ALL), non-Hodgkin's lymphoma (NHL), multiple myeloma (MM), bladder cancer, ovarian cancer, prostate cancer, lung cancer, colon cancer, breast cancer, pancreatic cancer, liver cancer, renal cell carcinoma, melanoma, glioblastomas, cervical cancer or angiosarcoma.

The disclosure also provides a method for treating other diseases associated with VEGF and/or PD-L1 signaling/over-expression using the pharmaceutical composition of the disclosure, including age-related macular degeneration (AMD), diabetic retinopathy, and liver fibrosis.

The present disclosure is now further described with the non-limiting examples below.

### EXAMPLES

The structures of the exemplary fusion proteins of the disclosure, IMM2518 and IMM2519, and the control proteins are briefly described below.

IMM2515H is an IgG antibody specifically binding PD-L1, comprising two heavy chains and two light chains, wherein the amino acid sequences of the heavy chain variable region, the heavy chain constant region, the light chain variable region and the light chain constant region are set forth in SEQ ID NOs: 7, 9, 8 and 10, respectively. The amino acid sequences of the heavy chain and the light chain are set forth in SEQ ID NOs: 15 and 14, respectively.

IMM2518 is an exemplary recombinant fusion protein of the disclosure, comprising two copies of VEGFR1D2 (SEQ ID NO: 11) linked via a linker (SEQ ID NO: 12) to the N-termini of the two heavy chains of IMM2515H, as shown in FIG. 1. In other words, IMM2518 comprises two long chains of SEQ ID NO: 13, and two short chains of SEQ ID NO: 14.

IMM2519 is an exemplary recombinant fusion protein of the disclosure, comprising two copies of VEGFR1D2 (SEQ ID NO: 11) linked via a linker (SEQ ID NO: 12) to the N-termini of the two light chains of IMM2515H, as shown in FIG. 1. In other words, IMM2519 comprises two long chains of SEQ ID NO: 15, and two short chains of SEQ ID NO: 16.

IMM2510 is a recombinant fusion protein having a similar structure to IMM2518, with only the anti-PD-L1 antibody being different, and specifically contains two long chains of SEQ ID NO: 17 and two short chains of SEQ ID NO: 18.

VEGFR1D2-Fc is a fusion protein consisting of VEGFR1D2 and Fc, and comprises the amino acid sequence of SEQ ID NO: 19.

### Example 1. Construction of vectors expressing recombinant fusion proteins

### 1.1 IMM2518

Full-length sequences encoding IMM2518 were designed artificially. Specifically, for the long chain, 57 nucleotides encoding the signal peptide of mouse IgG1 heavy chain (SEQ ID NO: 21) were added to the 5' end of the VEGF binding peptide-linker-anti-PD-L1 heavy chain-coding sequence (SEQ ID NO: 23), and a Kozak sequence (GCCGCCACC) was added to the 5' end of the signal peptide sequence. Lastly, HindIII and NheI restriction sites were added to the 5' and 3' ends of the resulting sequence, respectively. For the short chain, the same signal sequence and Kozac sequence were added to the coding sequence of the anti-PD-L1 light chain (SEQ ID NO: 24), and HindIII and the XbaI restriction sites were added to 5' and 3' ends of the resulting sequence, respectively. The two resulting sequences were artificially synthesized and subcloned, respectively, into the pMac-H and pMac-L vectors.

### 1.2 IMM2519

Full-length sequences encoding IMM2519 were designed artificially. Specifically, for the long chain, 57 nucleotides encoding the signal peptide of mouse IgG1 heavy chain (SEQ ID NO: 21) were added to the 5' end of the anti-PD-L1 heavy chain-coding sequence (SEQ ID NO: 25), and a Kozak sequence (GCCGCCACC) was added to the 5' end of the signal peptide sequence. Lastly, HindIII and NheI restriction sites were added to the 5' and 3' ends of the resulting sequence, respectively. For the short chain, the same signal sequence and Kozac sequence were added to the coding sequence of the VEGF binding peptide-linker-anti-PD-L1 light chain (SEQ ID NO: 26), and HindIII and the XbaI restriction sites were added to 5' and 3' ends of the resulting sequence, respectively. The two resulting sequences were artificially synthesized and subcloned, respectively, into the pMac-H and pMac-L vectors.

### Example 2. Preparation of recombinant fusion proteins and quality analysis

The expression vectors as constructed were used to transiently express proteins in CHO-S cells. Generally, 1) CHO-S cells at the density of 1 × 10⁶/ml were inoculated in transient transfection medium (TransFx-CTMCHO Transient Transfection Medium, Hyclone) containing 6 mM glutamine one day before transient transfection; 2) the heavy/long chain expression vectors and the light/short chain expression vectors, at a 1: 1 mass ratio, with 1 µg/ml DNA in total, were added to OPTI-MEM culture medium (Gibco) with a volume accounting 1/20 of the total transient transfection system; 3) PEI (MW 40,000 polyetherimide hydrochloride, polysciences) prepared at 1 mg/ml was added to OPTI-MEM culture medium (Gibco) at a volume accounting for 1/20 of the transient transfection system, at the PEI: DNA ratio of 4: 1; 4) the PEI dilution was slowly added to the DNA dilution, mixed and incubated at room temperature for 20 min; 5) the PEI/DNA mixture was added to the cell culture, and the cells were cultured in an incubator shanking at 110 rpm at 37°C, 5%CO₂; 6) an transfection enhancer (1 mM sodium butyrate, 0.25%V/V DMSO) was added two days later, and the temperature was decreased to 33°C for the culture; 7) the supernatants were collected with centrifugation at 3000 rpm for 5 min, when the cell viability decreased to 50% or lower, and subjected to affinity purification by Protein A.

The SEC-HPLC diagrams showed that IMM2518 had a relatively high purity, with relatively low aggregation amount and little degradation, as specified in Table 1.

**Table 1. SEC-HPLC results of recombinant fusion proteins of the disclosure**

| Sample | Aggregation (%) | Main peak (%) | Degradation (%) |
|---|---|---|---|
| IMM2518 | 4.76 | 94.98 | 0.26 |
| IMM2519 | 18.72 | 77.55 | 3.73 |

### Example 3. Recombinant fusion proteins bound to VEGF-165

For the VEGF binding assay, recombinant human VEGF-165 protein (Cat#11066-HNAH, Sino Biologicals) was prepared in carbonate-bicarbonate coating buffer (Cat#C3041, Sigma-Aldrich) and transferred to an ELISA plate, 50 ng/well, and the plate was placed in a refrigerator at 4°C overnight. The plate was blocked with 150 µL/well 3% skimmed milk at room temperature for 2 hr. The plate was then added with 100 µL serially diluted recombinant fusion proteins, incubated at 37°C for 1 hr, and washed for 5 times using PBS-T with 0.05% Tween 20. The plate was added with HRP-rabbit-anti-human IgG Fc (Cat#: 309-036-008, Jackson ImmunoResearchLab) and incubated at 37°C for 1 hr. The plate was washed using PBS-T for 5 times, added with 100 µL TMB, followed by 500 µL 1 N H₂SO₄ to stop color development, and measured for absorbance in a microplate reader.

As shown in FIG. 2, the two recombinant fusion proteins of the disclosure had similar VEGF-165 binding activity.

### Example 4. Recombinant fusion proteins bound to PD-L1

For the PD-L1 binding assay, 100 µl 1×10⁶/ml CHO-PD-L1 cells (Cat#YMAK-C006, ImmuneOnco) were incubated respectively with 100 µl serially diluted recombinant fusion proteins and control proteins, IMM2515H and hIgG1-Fc (SEQ ID NO: 20), at 4°C for 40 min. After washed with cold PBS, the cells were incubated with FITC-conjugated anti-human IgG-Fc antibodies (Cat#F9512, Sigma) for 40 min. The cells were then washed twice and subjected to FACS analysis.

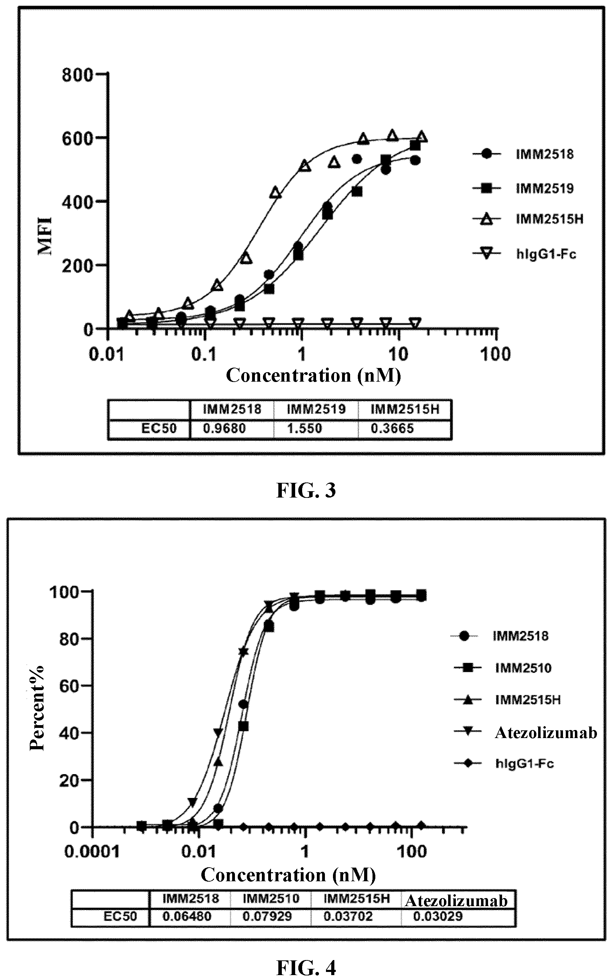

The results were shown in FIG. 3. IMM2518 and IMM2519's binding activities to PD-L1⁺ cells were 2.6-fold and 4.2-fold lower than that of the monospecific anti-PD-L1 antibody IMM2515H.

The recombinant fusion proteins were also tested for their binding activity to the CHO-PD-L1 cells along with the control proteins, IMM2515H, IMM2510, Atezolizumab and hIgG1-Fc, following the protocol above.

As shown in FIG. 4, IMM2518's binding activity to PD-L1⁺ cells was close to that of IMM2510.

### Example 5. Recombinant fusion protein's binding affinity to VEGF and PD-L1

The recombinant fusion protein's binding affinity to the antigens was tested in a label-free biomolecule analyzer (Access Medical Systems, Gator bio) in the Kinetics Assay mode, using the Q buffer (10 mM, PH7.4, PBS+0.02%Tween+0.2%BSA) as the assay buffer, and the human Fc probe as the assay probe, wherein IMM2518 was 2-fold diluted starting at 10 µg/ml (5 concentrations in total), and the antigens as used were 10 µg/ml VEGF165 (Cat#11066-HNAH, sino Biological) and 10 µg/ml PD-L1-his (Cat#90251-C08H, sino Biological). The probe was regenerated using a regeneration buffer, pH 2.0 (10 mM Gly+150 mM NaCl, PH 2.0), and the analyzer was set at 25°C.

**Table 2. Recombinant fusion protein's affinity to VEGF and PD-L1 as measured by BLI**

| Binding target | K_{off} (1/s) | Kₒₙ (1/ms) | K_{D} (M) |
|---|---|---|---|
| VEGF165 | NA | 1.57E+05 | NA |
| PD-L1-his | 0.000218 | 4.94E+05 | 4.41E-10 |

The test results showed that, the dissociation rate was very low when IMM2518 bound to VEGF165, resulting in no specific constant of dissociation. IMM2518's PD-L1 affinity was 0.441 nM.

### Example 6. Recombinant fusion protein's blocking activity on PD-1-PD-L1

For the analysis of the PD-1-PD-L1 blocking activity, 50 µl 5×10⁵/ml CHO-PD-L1 cells (Cat#YMAK-C006, ImmuneOnco) were incubated respectively with 50 µl serially diluted recombinant fusion proteins and control proteins (IMM2515H, IMM2510, Atezolizumab and hIgG1-Fc) at 4°C for 45 min, added with 50 µl PD1-mFc (in house made by ImmuneOnco, SEQ ID NO: 22), incubated at 4°C for 45 min. After washed with cold PBS, the cells were incubated with PE-conjugated anti-mouse IgG-Fc antibodies (Biolegend, Cat#405307). The cells were then washed twice and subjected to FACS analysis.

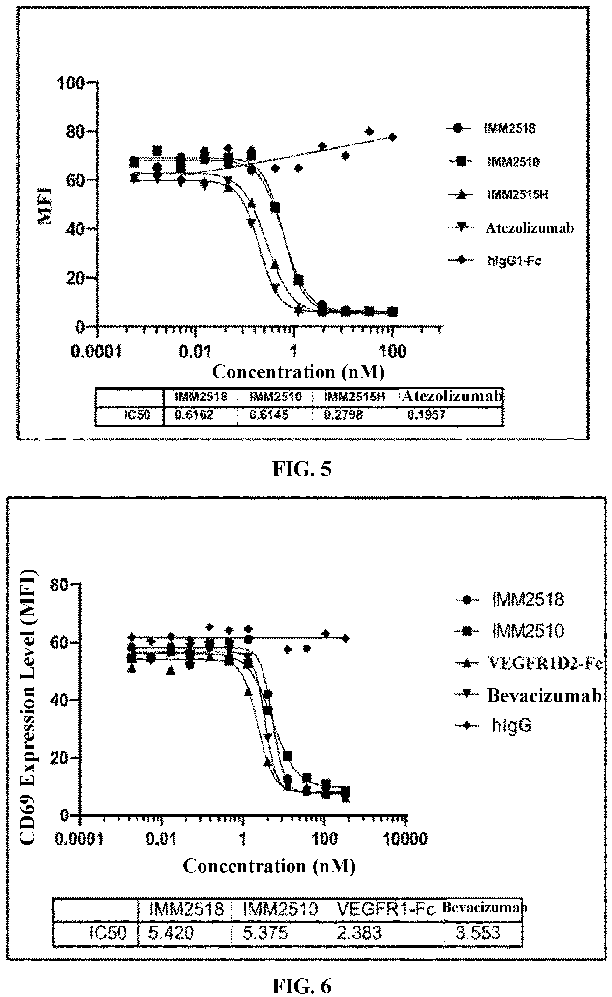

As shown in FIG. 5, the capability of IMM2518 to block the binding of the PD-1 protein to PD-L1⁺ cells was 2.2-fold and 3-fold lower than those of IMM2515H and atezolizumab, respectively, and close to that of IMM2510.

### Example 7. Recombinant fusion protein's blocking activity on VEGF activity

The blocking ability on VEGF activity was measured using Jurkat-CVR cells constructed by ImmuneOnco. The CVR (a chimeric VEGFR1D2 receptor), consisted of a second extracellular domain of VEGFR1, a CD8α hinge domain, a transmembrane region and an intracellular domain of CD28, and a CD3z signaling domain, contained the amino acid sequence of SEQ ID NO: 6. Fifty (50) µl 5×10⁵/ml Jurkat-CVR cells were incubated respectively with 50 µl serially diluted recombinant fusion proteins and control proteins (VEGFR1D2-Fc (SEQ ID NO: 19), IMM2510, Bevacizumab and hIgG1-Fc) at 37°C for 45 min, added with 50 µl VEGF-Fc (in house made by ImmuneOnco, SEQ ID NO: 27), incubated at 37°C overnight. After washed with cold PBS, the cells were incubated with PE-conjugated anti-human CD69 antibodies (Biolegend, Cat#310906). The cells were then washed twice and subjected to FACS analysis.

As shown in FIG. 6, the blocking ability of IMM2518 on VEGF activity was a bit lower than those of VEGFR1D2-Fc and Bevacizumab, and close to that of IMM2510.

### Example 8. Recombinant fusion protein's PD-L1 binding specificity

The human PD-L1 (Cat#10084-H08H, sino Biological), mouse PD-L1 (Cat#50010-M08H, sino Biological), monkey PD-L1 (Cat#90251-C08H, sino Biological), rat PD-L1 (Cat#80450-R08H, sino Biological), and human PD-L2 (Cat#10292-H08H, sino Biological) in carbonate-bicarbonate coating buffer (Cat#C3041, Sigma-Aldrich) were coated onto a plate, 50 ng/well, and the plate was placed in a refrigerator at 4°C overnight. The plate was blocked with 150 µL/well 3% skimmed milk at room temperature for 2 hr. The plate was added with 100 µL serially diluted recombinant fusion proteins, incubated at 37°C for 1 hr, and washed for 5 times using PBS-T with 0.05% Tween 20. The plate was added with HRP-rabbit-anti-human IgG Fc (Cat#: 309-036-008, Jackson ImmunoResearchLab) and incubated at room temperature for 1 hr. The plate was washed using PBS-T for 5 times, added with 100 µL TMB, followed by 500 µL 1 N H₂SO₄ to stop color development, and measured for absorbance in a microplate reader.

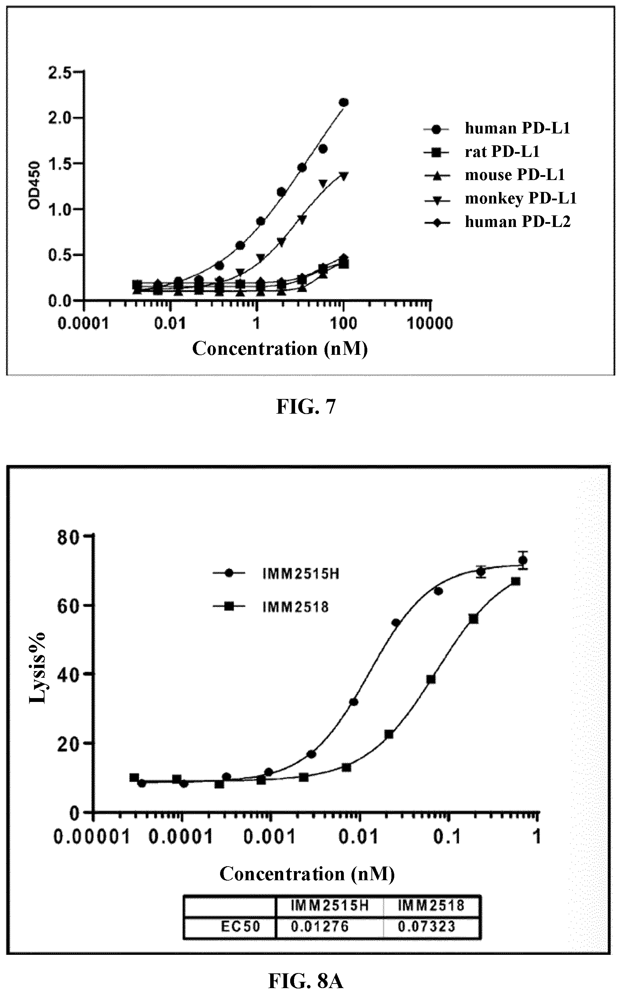

As shown in FIG. 7, IMM2518 bound to human PD-L1 and cross-reacted with monkey PD-L1, but did not cross-react with rat PD-L1, mouse PD-L1 or human PD-L2.

### Example 9. Recombinant fusion protein induced antibody-dependent cell mediated cytotoxicity (ADCC)

Fifty (50) µl 6×10⁵/ml Raji-PD-L1 cells labelled with CFSE dye (Cat#21888-25 mg, Sigma) and 100 µl 6×10⁵/ml NK92MI cells stably expressing FcγRIIIa (158V) (Cat#CRL-2408, ATCC) as the effector cells, were mixed at a 1: 2 ratio, and the mixed cells were cultured with 50 µl serially diluted IMM2518 or IMM2515H at 37°C for 4 hr. The cell culture was then added with propidium iodide (PI, Cat#P4170, Sigma) at the final concentration of 5 µg/ml, and subjected to FACS to measure the PI signals from the CFSE-labelled target cells. The ADCC-mediated cell lysis percent was calculated as %lysis (or %ADCC)=(%PI positive cells treated with IMM2518 or positive control-%PI positive cells treated by negative control protein)/(100-%PI positive cells treated by negative control protein)*100.

The results were shown in FIG. 8A. The ADCC induced by IMM2518 against Raji-PD-L1 cells was 6-fold lower than that induced by IMM2515H.

Fifty (50) µl 5×10⁵/ml RKO cells (Cat#TCHu116, Cell Collection of Chinese Academy of Sciences) or HCC827 cells (Cat#TCHu153, Cell Collection of Chinese Academy of Sciences) treated with trypsin, and 50 µl 5×10⁵/ml NK92MI cells stably expressing FcγRIIIa (158V) (Cat#CRL-2408, ATCC) as the effector cells, were mixed at a 1: 1 ratio, and the mixed cells were cultured with 50 µl serially diluted IMM2518, IMM2515H, IMM2510, Atezolizumab, or hIgG1-Fc at 37°C, 5% CO₂ overnight. The cell culture was then added with 20 µl CCK-8 (Beyotime, Cat# C0039), incubated in an incubator for 3 hr, and measured for absorbance at 450 nm. The ADCC-mediated cell lysis percent was calculated as %lysis (or %ADCC)=(OD450 of effector cells and target cells in negative control group-OD450 of effector cells and target cells treated by IMM2518 or positive control)/(OD450 of effector cells and target cells in negative control group-OD450 of effector cells in negative control group)*100%.

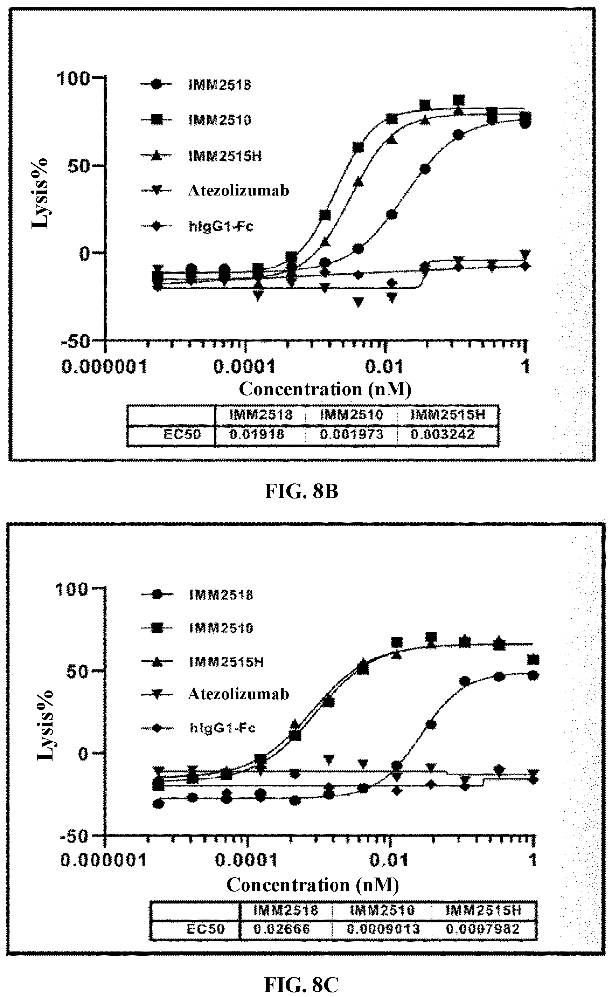

As shown in FIG. 8B and FIG. 8C, the ADCC induced by IMM2518 against RKO cells and HCC827 cells was 6-fold and 33-fold lower than that induced by IMM2515H, respectively.

### Example 10. Recombinant fusion protein induced antibody-dependent cellular phagocytosis (ADCP)

THP-1 cells (Cat#TCHu 57, Cell Collection of Chinese Academy of Sciences), 2.5×10⁵/ml, in 100 µl complete culture medium, were seeded onto a 96-well flat-bottom plate containing 200 ng/ml PMA (Sigma, Cat#379346-1MG) and cultured for 48 hr. The RKO cells (Cat#TCHu116, Cell Collection of Chinese Academy of Sciences), as the target cells, were treated by trypsin and labelled with CFSE (Cat#21888-25mg, Sigma). One hundred (100) µl 5.0×10⁵/ml CFSE-labelled RKO cells, were mixed with the THP-1 cells on the 96-well plate in an effector: target ratio of 1: 2, and the mixed cells were cultured with 50 µl serially diluted IMM2518, IMM2515H, IMM2510, Atezolizumab, or hIgG1-Fc at 37°C, 5%CO₂ for 2 hr. After the incubation, the supernatants were discarded, and the plate was washed for 5 times with 250 µl/well PBS, to remove the unbound target cells. The THP-1 cells were suspended by repeated blowing, and subjected to FACS to measure the CFSE signals from the THP-1 macrophages.

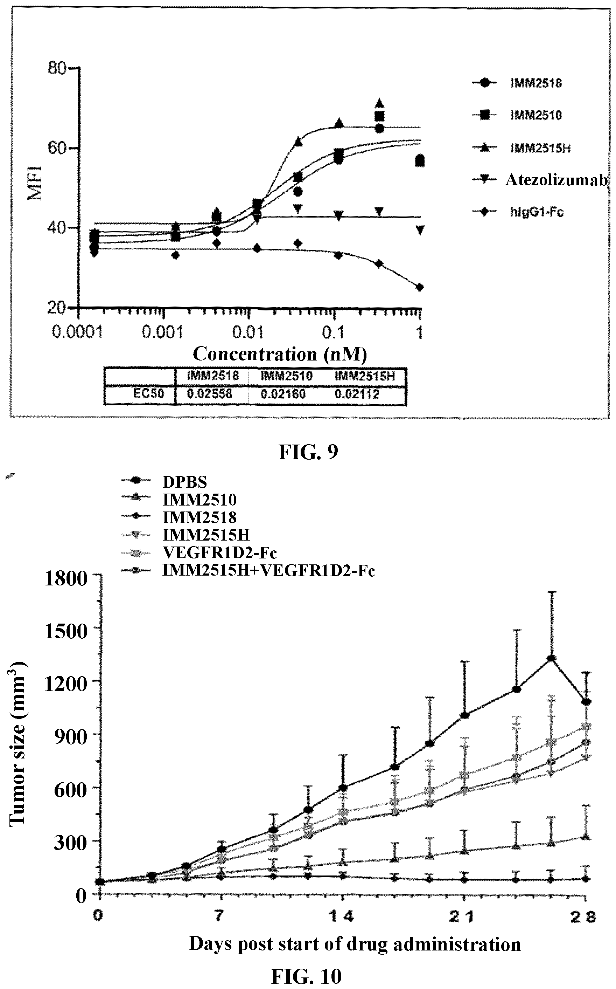

As shown in FIG. 9, the phagocytic activities induced by IMM2518, IMM2515H and IMM2510 against RKO tumor cells were close.

### Example 11. Recombinant fusion protein had good anti-tumor effect

The *in vivo* anti-tumor efficacy of the recombinant fusion protein of the disclosure was tested using humanized C57BL/6 hPD-1 mice (Gempharmtech). MC38-hPD-L1 cells (a mouse colon tumor cell line, AppTec) were *in vitro* monolayer cultured, and digested with trypsin-EDTA twice a week for cell passaging. When the cell saturation density reached 80% to 90% and the total count met the requirement, the cells were collected and counted.

The mice were subcutaneously inoculated with 3×10⁵ MC38-hPD-L1 cells in 100 µL PBS at the right axilla. Thirty-six (36) animals with the average tumor size of 60-90 mm³ were picked and randomly allocated into 6 groups, 6 mice each group, on Day 0. From this day on, the mice were intraperitoneally administered with DPBS, IMM2510 (6.0 mg/kg), IMM2518 (6.0 mg/kg), IMM2515H (5.0 mg/kg), VEGFR1D2-Fc (2.5 mg/kg), and IMM2515H (5.0 mg/kg) +VEGFR1D2-Fc (2.5 mg/kg) combination, respectively, for 4 weeks, twice a week. The tumor size and animal weight were measured twice a week.

The tumor volume (V) was calculated as (length × width²)/2. The tumor growth inhibition (TGI) was calculated by the formula: Tumor growth inhibition rate = (1-tumor volume change in administration group/tumor volume change in control group) ×100%.

The data were presented as Mean ± S.E.M. The comparison was done between two groups using Dunnett's Multiple Comparison, and a p-value less than 0.05 was considered to be statistically significant.

As shown in FIG. 10, as compared to the DPBS group, the mice treated with the monospecific targeted agents or the combination of two monospecific targeted agents had their tumor size increased slowly, and an evident slower tumor size increase was observed in mice treated with IMM2510 or IMM2518. The anti-tumor efficacy of IMM2518 was higher than that of IMM2510, wherein the TGI of IMM2510 treatment was 74.07% and the TGI of IMM2518 treatment was 97.47%.

### Example 12. Anti-tumor effects of recombinant fusion protein administered in different ways

Following the test steps of Example 11, the recombinant fusion protein was tested at a lower dose for its *in vivo* anti-tumor effect with different administration routes.

The humanized C57BL/6 hPD-1 mice were subcutaneously inoculated with 3×10⁵ MC38-hPD-L1 cells in 100 µL PBS at the right axilla. The animals with the average tumor size of 60-90 mm³ were picked and randomly allocated into 4 groups, 6 mice each group, on Day 0. From this day on, the mice were administered with DPBS via intraperitoneal (IP) injection, IMM2518 (4.0 mg/kg) via intraperitoneal (IP) injection, IMM2518 (4.0 mg/kg) via subcutaneous (SC) injection, and IMM2518 (4.0 mg/kg) via tail intravenous injection, respectively, for 4 weeks, twice a week. The tumor size and animal weight were measured twice a week.

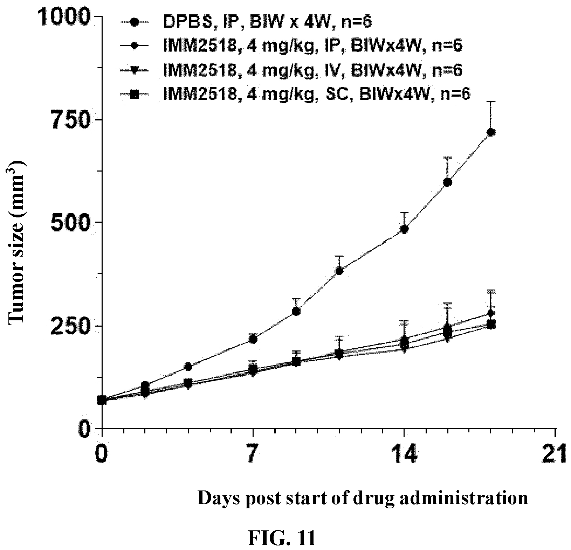

The mouse tumor size of each group during the test was shown in FIG. 11. It can be seen that, IMM2518, when administered at a relatively low dose, still significantly inhibited tumor growth, as compared to the DPBS group, and the effects were similar among the groups with intraperitoneal injection, subcutaneous injection and intravenous injection.

The sequences of the application were summarized below.

| Description/ Sequence/SEQ ID NO. |
|---|
| VH-CDR1 of IMM2515H |
| GYTFTSNW (SEQ ID NO: 1) |
| VH-CDR2 of IMM2515H |
| IHPNSGSS (SEQ ID NO: 2) |
| VH-CDR3 of IMM2515H |
| ARSYYGSSPYYFDY (SEQ ID NO: 3) |
| VL-CDR1 of IMM2515H |
| QDIINY (SEQ ID NO: 4) |
| VL-CDR2 of IMM2515H |
| YTS |
| VL-CDR3 of IMM2515H |
| QQGDTLPWT (SEQ ID NO: 5) |
| VH of IMM2515H |
| QVQLVQSGAEVKKPGASVKVSCKASGYTFTSNWMHWVRQAPGQGLEWMGMIH PNSGSSNYNEKFKSRVTMTRDTSTSTVYMELSSLRSEDTAVYYCARSYYGSSPYY |
| FDYWGQGTLVTVSS (SEQ ID NO: 7) |
| VL of IMM2515H |
| DIQMTQSPSSLSASVGDRVTITCRASQDIINYLNWYQQKPGKAPKLLIYYTSRLHS GVPSRFSGSGSGTDFTFTISSLQPEDIATYYCQQGDTLPWTFGQGTKVEIK (SEQ ID NO: 8) |
| Heavy chain constant region |
| ASTKGPSVFPLAPSSKSTSGGTAALGCLVKDYFPEPVTVSWNSGALTSGVHTFPAV LQSSGLYSLSSVVTVPSSSLGTQTYICNVNHKPSNTKVDKRVEPKSCDKTHTCPPC PAPELLGGPSVFLFPPKPKDTLMISRTPEVTCVVVDVSHEDPEVKFNWYVDGVEV HNAKTKPREEQYNATYRVVSVLTVLHQDWLNGKEYKCKVSNKALPAPIAATISK AKGQPREPQVYTLPPSREEMTKNQVSLTCLVKGFYPSDIAVEWESNGQPENNYKT TPPVLDSDGSFFLYSKLTVDKSRWQQGNVFSCSVMHEALHNHYTQKSLSLSPGK (SEQ ID NO: 9) |
| Light chain constant region |
|  (SEQ ID NO: 10) |
| VEGF binding peptide |
| SDTGRPFVEMYSEIPEIIHMTEGRELVIPCRVTSPNITVTLKKFPLDTLIPDGKRIIWD SRKGFIISAATYKEIGLLTCEATVNGHLYKTNYLTHRQTNT (SEQ ID NO: 11) |
| Linker |
| GGGGSGGGGSGGGGS (SEQ ID NO: 12) |
| IMM2518's long chain |
| SDTGRPFVEMYSEIPEIIHMTEGRELVIPCRVTSPNITVTLKKFPLDTLIPDGKRIIWD SRKGFIISAATYKEIGLLTCEATVNGHLYKTNYLTHRQTNTGGGGSGGGGSGGGGS QVQLVQSGAEVKKPGASVKVSCKASGYTFTSNWMHWVRQAPGQGLEWMGMIH PNSGSSNYNEKFKSRVTMTRDTSTSTVYMELSSLRSEDTAVYYCARSYYGSSPYY FDYWGQGTLVTVSSASTKGPSVFPLAPSSKSTSGGTAALGCLVKDYFPEPVTVSW NSGALTSGVHTFPAVLQSSGLYSLSSVVTVPSSSLGTQTYICNVNHKPSNTKVDKR VEPKSCDKTHTCPPCPAPELLGGPSVFLFPPKPKDTLMISRTPEVTCVVVDVSHED PEVKFNWYVDGVEVHNAKTKPREEQYNATYRVVSVLTVLHQDWLNGKEYKCK VSNKALPAPIAATISKAKGQPREPQVYTLPPSREEMTKNQVSLTCLVKGFYPSDIAV EWESNGQPENNYKTTPPVLDSDGSFFLYSKLTVDKSRWQQGNVFSCSVMHEALH NHYTQKSLSLSPGK (SEQ ID NO: 13) |
| Atgggatggtcatgtatcatcctttttctggtagcaactgcaactggagtacattcaagtgatacaggtagacctttcgtagagatgt |
| acagtgaaatccccgaaattatacacatgactgaaggaagggagctcgtcattccctgccgggttacgtcacctaacatcactgtt actttaaaaaagtttccacttgacactttgatccctgatggaaaacgcataatctgggacagtagaaagggcttcatcatatcagctg caacgtacaaagaaatagggcttctgacctgtgaagcaacagtcaatgggcatttgtataagacaaactatctcacacatcgacaa accaatacaggcggcggtgggagcggcggcggtgggagcggcggcgggggctcgCAAGTGCAGCTGGTGC AGAGCGGCGCCGAGGTGAAGAAGCCTGGCGCAAGCGTGAAGGTGAGCTGCA AGGCAAGCGGCTACACCTTCACAAGCAACTGGATGCACTGGGTGAGACAAGC CCCTGGCCAAGGCCTGGAGTGGATGGGCATGATCCACCCTAACAGCGGCAGCA GCAACTACAACGAGAAGTTCAAGAGCAGAGTGACCATGACAAGAGACACAA GCACAAGCACCGTGTACATGGAGCTGAGCAGCCTGAGAAGCGAGGACACCGC CGTGTACTACTGCGCTAGAAGCTACTACGGCAGCAGCCCTTACTACTTCGACTA CTGGGGCCAAGGCACCCTGGTGACCGTGAGCAGCGCTAGCACCAAGGGCCCA TCGGTCTTCCCCCTGGCACCCTCCTCCAAGAGCACCTCTGGGGGCACAGCGGC CCTGGGCTGCCTGGTCAAGGACTACTTCCCCGAACCGGTGACGGTGTCGTGGA ACTCAGGCGCCCTGACCAGCGGCGTGCACACCTTCCCGGCTGTCCTACAGTCC TCAGGACTCTACTCCCTCAGCAGCGTGGTGACCGTGCCCTCCAGCAGCTTGGG CACCCAGACCTACATCTGCAACGTGAATCACAAGCCCAGCAACACCAAGGTG GACAAGAGAGTTGAGCCCAAATCTTGTGACAAAACTCACACATGCCCACCGT GCCCAGCACCTGAACTCCTGGGGGGACCGTCAGTCTTCCTCTTCCCCCCAAAA CCCAAGGACACCCTCATGATCTCCCGGACCCCTGAGGTCACATGCGTGGTGGT GGACGTGAGCCACGAAGACCCTGAGGTCAAGTTCAACTGGTATGTGGACGGC GTGGAGGTGCATAATGCCAAGACAAAGCCGCGGGAGGAGCAGTACAACGCCA CGTACCGTGTGGTCAGCGTCCTCACCGTCCTGCACCAAGACTGGCTGAATGGC AAGGAGTACAAGTGCAAGGTCTCCAACAAAGCCCTCCCAGCCCCCATCGCCG CAACCATCTCCAAAGCCAAAGGGCAGCCCCGAGAACCACAGGTGTACACCCT GCCCCCATCCCGGGAGGAGATGACCAAGAACCAAGTCAGCCTGACCTGCCTG GTCAAAGGCTTCTATCCCAGCGACATCGCCGTGGAGTGGGAGAGCAATGGGCA GCCGGAGAACAACTACAAGACCACGCCTCCCGTGCTGGACTCCGACGGCTCC TTCTTCCTCTATTCCAAGCTCACCGTGGACAAGAGCAGGTGGCAGCAGGGGAA CGTCTTCTCATGCTCCGTGATGCATGAGGCTCTGCACAACCACTACACGCAGA AGAGCCTCTCCCTGTCTCCGGGCAAATGA (SEQ ID NO: 23) |
| IMM2518's short chain |
| DIQMTQSPSSLSASVGDRVTITCRASQDIINYLNWYQQKPGKAPKLLIYYTSRLHS GVPSRFSGSGSGTDFTFTISSLQPEDIATYYCQQGDTLPWTFGQGTKVEIKRTVAAP SVFIFPPSDEQLKSGTASVVCLLNNFYPREAKVQWKVDNALQSGNSQESVTEQDS KDSTYSLSSTLTLSKADYEKHKVYACEVTHQGLSSPVTKSFNRGEC (SEQ ID NO: 14) |
| ATGGGATGGTCATGTATCATCCTTTTTCTGGTAGCAACTGCAACTGGAGTACATT CAGACATTCAGATGACACAGAGCCCTAGCAGCCTGAGCGCAAGCGTGGGCGA CAGAGTGACCATCACCTGCAGAGCAAGCCAAGACATCATCAACTACCTGAACT GGTATCAGCAGAAGCCTGGCAAGGCCCCTAAGCTGCTGATCTACTACACAAGC AGACTGCACAGCGGCGTGCCTAGCAGATTCAGCGGCAGCGGCAGCGGCACCG ACTTCACCTTCACCATCAGCAGCCTGCAGCCTGAGGACATCGCCACCTACTAC TGTCAGCAAGGCGACACCCTGCCTTGGACCTTCGGCCAAGGCACCAAGGTGG AGATCAAGCGTGAGTTCTAGAGGATCCATCTGGGATAAGCATGCTGTTTTCTGT CTGTCCCTAACATGCCCTGTGATTATCCGCAAACAACACACCCAAGGGCAGAA CTTTGTTACTTAAACACCATCCTGTTTGCTTCTTTCCTCAGGAACTGTGGCTGC ACCATCTGTCTTCATCTTCCCGCCATCTGATGAGCAGTTGAAATCTGGAACTGC CTCTGTTGTGTGCCTGCTGAATAACTTCTATCCCAGAGAGGCCAAAGTACAGTG GAAGGTGGATAACGCCCTCCAATCGGGTAACTCCCAGGAGAGTGTCACAGAG CAGGACAGCAAGGACAGCACCTACAGCCTCAGCAGCACCCTGACGCTGAGCA AAGCAGACTACGAGAAACACAAAGTCTACGCCTGCGAAGTCACCCATCAGGG CCTGAGCTCGCCCGTCACAAAGAGCTTCAACAGGGGAGAGTGTTAG (SEQ ID NO: 24) |
| IMM2519's long chain |
| QVQLVQSGAEVKKPGASVKVSCKASGYTFTSNWMHWVRQAPGQGLEWMGMIH PNSGSSNYNEKFKSRVTMTRDTSTSTVYMELSSLRSEDTAVYYCARSYYGSSPYY FDYWGQGTLVTVSSASTKGPSVFPLAPSSKSTSGGTAALGCLVKDYFPEPVTVSW NSGALTSGVHTFPAVLQSSGLYSLSSVVTVPSSSLGTQTYICNVNHKPSNTKVDKR VEPKSCDKTHTCPPCPAPELLGGPSVFLFPPKPKDTLMISRTPEVTCVVVDVSHED PEVKFNWYVDGVEVHNAKTKPREEQYNATYRVVSVLTVLHQDWLNGKEYKCK VSNKALPAPIAATISKAKGQPREPQVYTLPPSREEMTKNQVSLTCLVKGFYPSDIAV EWESNGQPENNYKTTPPVLDSDGSFFLYSKLTVDKSRWQQGNVFSCSVMHEALH NHYTQKSLSLSPGK (SEQ ID NO: 15) |
| AtgggatggtcatgtatcatcctttttctggtagcaactgcaactggagtacattcaCAAGTGCAGCTGGTGCAG AGCGGCGCCGAGGTGAAGAAGCCTGGCGCAAGCGTGAAGGTGAGCTGCAAG GCAAGCGGCTACACCTTCACAAGCAACTGGATGCACTGGGTGAGACAAGCCC CTGGCCAAGGCCTGGAGTGGATGGGCATGATCCACCCTAACAGCGGCAGCAG CAACTACAACGAGAAGTTCAAGAGCAGAGTGACCATGACAAGAGACACAAGC ACAAGCACCGTGTACATGGAGCTGAGCAGCCTGAGAAGCGAGGACACCGCCG TGTACTACTGCGCTAGAAGCTACTACGGCAGCAGCCCTTACTACTTCGACTACT GGGGCCAAGGCACCCTGGTGACCGTGAGCAGCGCTAGCACCAAGGGCCCATC GGTCTTCCCCCTGGCACCCTCCTCCAAGAGCACCTCTGGGGGCACAGCGGCCC |
| TGGGCTGCCTGGTCAAGGACTACTTCCCCGAACCGGTGACGGTGTCGTGGAAC TCAGGCGCCCTGACCAGCGGCGTGCACACCTTCCCGGCTGTCCTACAGTCCTC AGGACTCTACTCCCTCAGCAGCGTGGTGACCGTGCCCTCCAGCAGCTTGGGCA CCCAGACCTACATCTGCAACGTGAATCACAAGCCCAGCAACACCAAGGTGGA CAAGAGAGTTGAGCCCAAATCTTGTGACAAAACTCACACATGCCCACCGTGCC CAGCACCTGAACTCCTGGGGGGACCGTCAGTCTTCCTCTTCCCCCCAAAACCC AAGGACACCCTCATGATCTCCCGGACCCCTGAGGTCACATGCGTGGTGGTGGA CGTGAGCCACGAAGACCCTGAGGTCAAGTTCAACTGGTATGTGGACGGCGTG GAGGTGCATAATGCCAAGACAAAGCCGCGGGAGGAGCAGTACAACGCCACGT ACCGTGTGGTCAGCGTCCTCACCGTCCTGCACCAAGACTGGCTGAATGGCAAG GAGTACAAGTGCAAGGTCTCCAACAAAGCCCTCCCAGCCCCCATCGCCGCAA CCATCTCCAAAGCCAAAGGGCAGCCCCGAGAACCACAGGTGTACACCCTGCC CCCATCCCGGGAGGAGATGACCAAGAACCAAGTCAGCCTGACCTGCCTGGTC AAAGGCTTCTATCCCAGCGACATCGCCGTGGAGTGGGAGAGCAATGGGCAGC CGGAGAACAACTACAAGACCACGCCTCCCGTGCTGGACTCCGACGGCTCCTTC TTCCTCTATTCCAAGCTCACCGTGGACAAGAGCAGGTGGCAGCAGGGGAACG TCTTCTCATGCTCCGTGATGCATGAGGCTCTGCACAACCACTACACGCAGAAG AGCCTCTCCCTGTCTCCGGGCAAATGA (SEQ ID NO: 25) |
| IMM2519's short chain |
| SDTGRPFVEMYSEIPEIIHMTEGRELVIPCRVTSPNITVTLKKFPLDTLIPDGKRIIW DSRKGFIISAATYKEIGLLTCEATVNGHLYKTNYLTHRQTNTGGGGSGGGGSGGG GSDIQMTQSPSSLSASVGDRVTITCRASQDIINYLNWYQQKPGKAPKLLIYYTSRL HSGVPSRFSGSGSGTDFTFTISSLQPEDIATYYCQQGDTLPWTFGQGTKVEIKRTV AAPSVFIFPPSDEQLKSGTASVVCLLNNFYPREAKVQWKVDNALQSGNSQESVTE QDSKDSTYSLSSTLTLSKADYEKHKVYACEVTHQGLSSPVTKSFNRGEC (SEQ ID NO: 16) |
| ATGGGATGGTCATGTATCATCCTTTTTCTGGTAGCAACTGCAACTGGAGTACATT CAagtgatacaggtagacctttcgtagagatgtacagtgaaatccccgaaattatacacatgactgaaggaagggagctcgtca ttccctgccgggttacgtcacctaacatcactgttactttaaaaaagtttccacttgacactttgatccctgatggaaaacgcataatct gggacagtagaaagggcttcatcatatcagctgcaacgtacaaagaaatagggcttctgacctgtgaagcaacagtcaatgggc atttgtataagacaaactatctcacacatcgacaaaccaatacaggcggcggtgggagcggcggcggtgggagcggcggcgg gggctcgGACATTCAGATGACACAGAGCCCTAGCAGCCTGAGCGCAAGCGTGGGC GACAGAGTGACCATCACCTGCAGAGCAAGCCAAGACATCATCAACTACCTGA ACTGGTATCAGCAGAAGCCTGGCAAGGCCCCTAAGCTGCTGATCTACTACACA AGCAGACTGCACAGCGGCGTGCCTAGCAGATTCAGCGGCAGCGGCAGCGGCA CCGACTTCACCTTCACCATCAGCAGCCTGCAGCCTGAGGACATCGCCACCTAC |
| TACTGTCAGCAAGGCGACACCCTGCCTTGGACCTTCGGCCAAGGCACCAAGG TGGAGATCAAGCGTGAGTTCTAGAGGATCCATCTGGGATAAGCATGCTGTTTTC TGTCTGTCCCTAACATGCCCTGTGATTATCCGCAAACAACACACCCAAGGGCA GAACTTTGTTACTTAAACACCATCCTGTTTGCTTCTTTCCTCAGGAACTGTGGC TGCACCATCTGTCTTCATCTTCCCGCCATCTGATGAGCAGTTGAAATCTGGAAC TGCCTCTGTTGTGTGCCTGCTGAATAACTTCTATCCCAGAGAGGCCAAAGTACA GTGGAAGGTGGATAACGCCCTCCAATCGGGTAACTCCCAGGAGAGTGTCACA GAGCAGGACAGCAAGGACAGCACCTACAGCCTCAGCAGCACCCTGACGCTGA GCAAAGCAGACTACGAGAAACACAAAGTCTACGCCTGCGAAGTCACCCATCA GGGCCTGAGCTCGCCCGTCACAAAGAGCTTCAACAGGGGAGAGTGTTAG (SEQ ID NO: 26) |
| IMM2515H's heavy chain |
| QVQLVQSGAEVKKPGASVKVSCKASGYTFTSNWMHWVRQAPGQGLEWMGMIH PNSGSSNYNEKFKSRVTMTRDTSTSTVYMELSSLRSEDTAVYYCARSYYGSSPYY FDYWGQGTLVTVSSASTKGPSVFPLAPSSKSTSGGTAALGCLVKDYFPEPVTVSW NSGALTSGVHTFPAVLQSSGLYSLSSVVTVPSSSLGTQTYICNVNHKPSNTKVDKR VEPKSCDKTHTCPPCPAPELLGGPSVFLFPPKPKDTLMISRTPEVTCVVVDVSHED PEVKFNWYVDGVEVHNAKTKPREEQYNATYRVVSVLTVLHQDWLNGKEYKCK VSNKALPAPIAATISKAKGQPREPQVYTLPPSREEMTKNQVSLTCLVKGFYPSDIAV EWESNGQPENNYKTTPPVLDSDGSFFLYSKLTVDKSRWQQGNVFSCSVMHEALH NHYTQKSLSLSPGK (SEQ ID NO: 15) |
| IMM2515H's short chain |
| DIQMTQSPSSLSASVGDRVTITCRASQDIINYLNWYQQKPGKAPKLLIYYTSRLHS GVPSRFSGSGSGTDFTFTISSLQPEDIATYYCQQGDTLPWTFGQGTKVEIKRTVAAP SVFIFPPSDEQLKSGTASVVCLLNNFYPREAKVQWKVDNALQSGNSQESVTEQDS KDSTYSLSSTLTLSKADYEKHKVYACEVTHQGLSSPVTKSFNRGEC (SEQ ID NO: 14) |
| IMM2510's long chain |
| SDTGRPFVEMYSEIPEIIHMTEGRELVIPCRVTSPNITVTLKKFPLDTLIPDGKRIIWD SRKGFIISAATYKEIGLLTCEATVNGHLYKTNYLTHRQTNTGGGGSGGGGSGGGGS EVQLVESGGGLVQPGGSLRLSCAASGFTFSDSWIHWVRQAPGKGLEWVAWISPY GGSTYYADSVKGRFTISADTSKNTAYLQMNSLRAEDTAVYYCARRHWPGGFDY WGQGTLVTVSSASTKGPSVFPLAPSSKSTSGGTAALGCLVKDYFPEPVTVSWNSG ALTSGVHTFPAVLQSSGLYSLSSVVTVPSSSLGTQTYICNVNHKPSNTKVDKRVEP KSCDKTHTCPPCPAPELLGGPSVFLFPPKPKDTLMISRTPEVTCVVVDVSHEDPEV |
| KFNWYVDGVEVHNAKTKPREEQYNATYRVVSVLTVLHQDWLNGKEYKCKVSN KALPAPIAATISKAKGQPREPQVYTLPPSREEMTKNQVSLTCLVKGFYPSDIAVEW ESNGQPENNYKTTPPVLDSDGSFFLYSKLTVDKSRWQQGNVFSCSVMHEALHNH YTQKSLSLSPGK (SEQ ID NO: 17) |
| IMM2510's short chain |
| DIQMTQSPSSLSASVGDRVTITCRASQDVSTAVAWYQQKPGKAPKLLIYSASFLYS GVPSRFSGSGSGTDFTLTISSLQPEDFATYYCQQYLYHPATFGQGTKVEIKRTVAAP SVFIFPPSDEQLKSGTASVVCLLNNFYPREAKVQWKVDNALQSGNSQESVTEQDS KDSTYSLSSTLTLSKADYEKHKVYACEVTHQGLSSPVTKSFNRGEC (SEQ ID NO: 18) |
| VEGFR1D2-Fc |
| SDTGRPFVEMYSEIPEIIHMTEGRELVIPCRVTSPNITVTLKKFPLDTLIPDGKRIIWD SRKGFIISAATYKEIGLLTCEATVNGHLYKTNYLTHRQTNTEFEPKSCDKTHTCPPC PAPELLGGPSVFLFPPKPKDTLMISRTPEVTCVVVDVSHEDPEVKFNWYVDGVEV HNAKTKPREEQYNSTYRVVSVLTVLHQDWLNGKEYKCKVSNKALPAPIEKTISK AKGQPREPQVYTLPPSRDELTKNQVSLTCLVKGFYPSDIAVEWESNGQPENNYKT TPPVLDSDGSFFLYSKLTVDKSRWQQGNVFSCSVMHEALHNHYTQKSLSLSPGK (SEQ ID NO: 19) |
| hIgG1-Fc |
| EPKSCDKTHTCPPCPAPELLGGPSVFLFPPKPKDTLMISRTPEVTCVVVDVSHEDPE VKFNWYVDGVEVHNAKTKPREEQYNSTYRVVSVLTVLHQDWLNGKEYKCKVS NKALPAPIEKTISKAKGQPREPQVYTLPPSRDELTKNQVSLTCLVKGFYPSDIAVEW ESNGQPENNYKTTPPVLDSDGSFFLYSKLTVDKSRWQQGNVFSCSVMHEALHNH YTQKSLSLSPGK (SEQ ID NO: 20) |
| Mouse IgG1 heavy chain signal peptide |
| ATGGGATGGTCATGTATCATCCTTTTTCTGGTAGCAACTGCAACTGGAGTACATT CA (SEQ ID NO: 21) |
| Kozak sequence |
| GCCGCCACC |
| VEGF-Fc |
| MGWSCIILFLVATATGVHSAPMAEGGGQNHHEVVKFMDVYQRSYCHPIETLVDIF QEYPDEIEYIFKPSCVPLMRCGGCCNDEGLECVPTEESNITMQIMRIKPHQGQHIG EMSFLQHNKCECRPKKDRARQENPCGPCSERRKHLFVQDPQTCKCSCKNTDSRC KARQLELNERTCRCDKPRREFEPKSCDKTHTCPPCPAPELLGGPSVFLFPPKPKDT LMISRTPEVTCVVVDVSHEDPEVKFNWYVDGVEVHNAKTKPREEQYNSTYRVVS |
| VLTVLHQDWLNGKEYKCKVSNKALPAPIEKTISKAKGQPREPQVYTLPPSRDELT KNQVSLTCLVKGFYPSDIAVEWESNGQPENNYKTTPPVLDSDGSFFLYSKLTVDKS RWQQGNVFSCSVMHEALHNHYTQKSLSLSPGK (SEQ ID NO: 27) |
| PD1-mFc |
| MGWSCIILFLVATATGVHSLDSPDRPWNPPTFSPALLVVTEGDAATFTCSFSNTSES FVLNWYRMSPSNQTDKLAAFPEDRSQPGQDCRFRVTQLPNGRDFHMSVVRARR NDSGTYLCGAISLAPKAQIKESLRAELRVTERRAEVPTAHPSPSPRPAGQFQEFVPR DCGCKPCICTVPEVSSVFIFPPKPKDVLTITLTPKVTCVVVDISKDDPEVQFSWFVD DVEVHTAQTQPREEQFNSTFRSVSELPIMHQDWLNGKEFKCRVNSAAFPAPIEKTI SKTKGRPKAPQVYTIPPPKEQMAKDKVSLTCMITDFFPEDITVEWQWNGQPAENY KNTQPIMNTNGSYFVYSKLNVQKSNWEAGNTFTCSVLHEGLHNHHTEKSLSHSP GK (SEQ ID NO: 22) |
| CVR |
| MGWSCIILFLVATATGVHSSDTGRPFVEMYSEIPEIIHMTEGRELVIPCRVTSPNITVT LKKFPLDTLIPDGKRIIWDSRKGFIISNATYKEIGLLTCEATVNGHLYKTNYLTHRQ TNTTTTPAPRPPTPAPTIASQPLSLRPEACRPAAGGAVHTRGLDFACDFWVLVVVG GVLACYSLLVTVAFIIFWVRSKRSRLLHSDYMNMTPRRPGPTRKHYQPYAPPRDF AAYRSRVKFSRSAEPPAYQQGQNQLYNELNLGRREEYDVLDKRRGRDPEMGGKP RRKNPQEGLYNELQKDKMAEAYSEIGMKGERRRGKGHDGLYQGLSTATKDTYD ALHMQALPPR (SEQ ID NO: 6) |

While the application has been described above in connection with one or more embodiments, it should be understood that the application is not limited to those embodiments, and the description is intended to cover all alternatives and equivalents, as may be included within the spirit and scope of the appended claims. All references cited herein are further incorporated by reference in their entirety.

## Claims

1. A recombinant fusion protein, comprising an anti-PD-L1 antibody or an antigen-binding fragment thereof, and an VEGF binding peptide,
wherein the anti-PD-L1 antibody or the antigen-binding fragment thereof comprises a heavy chain variable region, a heavy chain constant region, and a light chain variable region, wherein the heavy chain variable region comprises a HV-CDR1, a HV-CDR2, and a HV-CDR3, wherein the light chain variable region comprises a LV-CDR1, a LV-CDR2, and a LV-CDR3, wherein the HV-CDR1, the HV-CDR2, the HV-CDR3, the LV-CDR1, and the LV-CDR3 comprise the amino acid sequences of SEQ ID NO: 1, 2, 3, 4 and 5, respectively, and the LV-CDR2 comprises the amino acid sequence "YTS", wherein the heavy chain constant region is linked to C-terminus of the heavy chain variable region and comprises FcR binding ability,
wherein the VEGF binding peptide comprises a second extracellular Ig-like domain of vascular endothelial growth factor receptor 1 (VEGFR1) (VEGFR1D2),
wherein the VEGF binding peptide is linked to N-terminus of the heavy chain variable region or the light chain variable region of the anti-PD-L1 antibody or the antigen-binding fragment thereof.

2. The recombinant fusion protein of claim 1, wherein the VEGF binding peptide comprises the amino acid sequence of SEQ ID NO: 11.

3. The recombinant fusion protein of claim 1, wherein the heavy chain variable region comprises the amino acid sequence of SEQ ID NO: 7, and the light chain variable region comprises the amino acid sequence of SEQ ID NO: 8.

4. The recombinant fusion protein of claim 1, wherein the VEGF binding peptide is linked to N-terminus of the heavy chain variable region of the anti-PD-L1 antibody or the antigen-binding fragment thereof.

5. The recombinant fusion protein of claim 1, wherein the heavy chain constant region comprises the amino acid sequence of SEQ ID NO: 9.

6. The recombinant fusion protein of claim 1, wherein the VEGF binding peptide is linked, via a linker, to the anti-PD-L1 antibody or the antigen-binding fragment thereof.

7. The recombinant fusion protein of claim 6, wherein the linker is -(Gly-Gly-Gly-Gly-Ser)₃- (SEQ ID NO: 12).

8. The recombinant fusion protein of claim 1, further comprising a light chain constant region, wherein the light chain constant region comprises the amino acid sequence of SEQ ID NO: 10 and is linked to C-terminus of the light chain variable region.

9. The recombinant fusion protein of claim 1, comprising:
i) a chain of VEGF binding peptide-linker-anti-PD-L1 antibody heavy chain variable region-heavy chain constant region that comprises the amino acid sequence of SEQ ID NO: 13, and a chain of anti-PD-L1 antibody light chain variable region-light chain constant region that comprises the amino acid sequence of SEQ ID NO: 14; or
ii) a chain of anti-PD-L1 antibody heavy chain variable region-heavy chain constant region that comprises the amino acid sequence of SEQ ID NO: 15, and a chain of VEGF binding peptide-linker-anti-PD-L1 antibody light chain variable region-light chain constant region that comprises the amino acid sequence of SEQ ID NO: 16.

10. A nucleic acid molecule, encoding the recombinant fusion protein of any one of claims 1 to 9.

11. An expression vector, comprising the nucleic acid molecule of claim 10.

12. A host cell, comprising the expression vector of claim 11, or comprising the nucleic acid molecule of claim 10 integrated into its genome.

13. A pharmaceutical composition, comprising the recombinant fusion protein of any one of claims 1 to 9, the nucleic acid molecule of claim 10, the expression vector of claim 11, or the host cell of claim 12, and at least one pharmaceutically acceptable carrier.

14. Use of the pharmaceutical composition of claim 13 in manufacture of a medicament for treating a disease associated with PD-L1 and/or VEGF signaling, wherein the disease is cancer, age-related macular degeneration (AMD), diabetic retinopathy, or hepatic fibrosis.

15. The use of claim 14, wherein the cancer is selected from the group consisting of acute myeloid leukemia (AML), chronic myeloid leukemia (CML), acute lymphoblastic leukemia (ALL), non-Hodgkin lymphoma (NHL), multiple myeloma (MM), bladder cancer, ovarian cancer, prostate cancer, lung cancer, colon cancer, breast cancer, pancreatic cancer, liver cancer, renal cell carcinoma, melanoma, glioblastoma multiforme, cervical cancer, and angiosarcoma.
